# EUROPEAN PATENT APPLICATION

(11) **EP 4 331 602 A1**
(43) Date of publication of application: **06.03.2024**
(21) Application number: 22193939.0
(22) Date of filing: 05.09.2022
(51) Int. Cl.: A61K 39/12, A61P 31/14

(54) **A LIVE ATTENUATED SARS-COV-2 AND A VACCINE MADE THEREOF**

(71) Applicant: Freie Universität Berlin, 14195 Berlin (DE)
(72) Inventor: TRIMPERT, Jakob, 10115 Berlin (DE); KUNEC, Dusan, 10551 Berlin (DE); OSTERRIEDER, Nikolaus, 14482 Potsdam (DE)
(74) Representative: Maikowski & Ninnemann Patentanwälte Partnerschaft mbB

(57) **Abstract**

The invention relates to a polynucleotide encoding a) severe acute respiratory syndrome coronavirus 2 (SARS-CoV-2) spike protein; and/or b) at least one non-structural SARS-CoV-2 protein selected from the group consisting of non-structural protein 7, non-structural protein 8, non-structural protein 9, non-structural protein 10, non-structural protein 11, non-structural protein 12, an endoribonuclease, and a 2'-O-methyltransferase, wherein the polynucleotide comprises or consists of at least one sequence part comprising codon-pair deoptimizations in comparison to the SARS-CoV-2 genome, and wherein the polynucleotide further comprises a furin cleavage site modification resulting in a loss of a furin cleavage site being naturally present in the SARS-CoV-2 genome. The invention further relates to a live attenuated SARS-CoV-2 comprising this polynucleotide, to a vaccine comprising this live attenuated SARS-CoV-2, as well as to associated methods.

## Description

The present invention relates to a codon-pair deoptimized polynucleotide encoding a respiratory syndrome coronavirus 2 (SARS-CoV-2) protein, to a live attenuated SARS-CoV-2 comprising such polynucleotide, to a pharmaceutical composition comprising such a live attenuated SARS-CoV-2, to a vaccination method for administering this pharmaceutical composition, to a vector comprising such polynucleotide, to a host cell comprising such polynucleotide, as well as to method of producing a virus.

Severe acute respiratory syndrome coronavirus 2 (SARS-CoV-2) emerged in December 2019 as the causative agent of coronavirus disease 2019 (COVID-19) (Wu et al., 2020; Zhou et al., 2020b). The virus is highly transmissible among humans (Chan et al., 2020). It has spread rapidly around the world within a matter of weeks and the world is still battling with the ongoing COVID-19 pandemic.

SARS-CoV-2 primarily replicates in the upper respiratory tract (Zou et al., 2020). The infection with SARS-CoV-2 can cause a wide spectrum of clinical manifestations, ranging from asymptomatic to life-threatening disease conditions (Chen et al., 2020; Zhou et al., 2020a). Especially the elderly and patients with pre-existing conditions are at greater risk of developing more severe disease such as pneumonia, acute respiratory distress syndrome and multiple organ failure (Chen et al., 2020; Garg et al., 2020; Zhou et al., 2020a). The ongoing pandemic imposes an enormous health, psychological, economic, and social burden. To date (December 2021) more than 270 million people have been infected with the virus, of whom more than 5.3 million have died as a result of the infection (https://coronavirus.jhu.edu/map.html) (Dong et al., 2020).

The unprecedented scale and severity of the COVID-19 pandemic prompted the rapid development of novel diagnostic tests, therapeutics and vaccines which could be used to contain the spread of the virus and limit the pandemic. Globally, more than 90 vaccines are being tested in clinical trials, but only few have reached the final stages of testing (Zimmer et al., 2021). Almost all vaccines that have been or are being evaluated in clinical trials are based either on inactivated or subunit virus preparations (Ella et al., 2021; Gao et al., 2020; Wang et al., 2020; Zhang et al., 2021), replication-defective virus vectors (Emary et al., 2021; Logunov et al., 2021; Solforosi et al., 2021; Voysey et al., 2021; Zhu et al., 2020), or DNA/RNA molecules (Anderson et al., 2020; Baden et al., 2021; Corbett et al., 2020; Dagan et al., 2021; Jackson et al., 2020; Mulligan et al., 2020; Polack et al., 2020; Sahin et al., 2020; Walsh et al., 2020).

SARS-CoV-2 is rapidly evolving (Tegally et al., 2021; Faria et al., 2021; Davies et al., 2021). Benefiting from its global presence, the virus continues to adapt to its new host and to infection-or vaccine-induced immunity. During the course of the pandemic, a number of genetic variants have emerged (Tegally et al., 2021; Faria et al., 2021; Davies et al., 2021). Variants that exhibit increased infectivity, cause greater morbidity and mortality, or have the ability to evade infection- or vaccine-induced immunity pose an increased threat to public health. The World Health Organization (WHO) and other national health agencies have independently established classification systems that categorize emerging variants as variants of interest (VOIs), variants under investigation (VUls), or variants of concern (VOCs) based on their risk to public health (cf. Table 1 of Trimpert et al. "Live attenuated virus vaccine protects against SARS-CoV-2 variants of concern B.1.1.7 (Alpha) and B.1.351 (Beta)", Science Advances, Vol. 7, No. 49 (2021)). In addition, to simplify communication with the public, the WHO recommends that VOIs and VOCs should also be labeled using the letters of the Greek alphabet. As of 12 August 2021, viruses belonging to lineages B.1.1.7 (Alpha), B.1.351 (Beta), B.1.1.28.1 (Gamma), B.1.617.2 (Delta), and, most recently, B.1.159.1 (Omicron) are classified by several health agencies as VOCs. In countries where they emerged, these variants rapidly supplanted the preexisting variants and started to spread globally.

The B.1.1.7 variant, first detected in the United Kingdom in December 2020, is 50 to 100% more transmissible and possibly also more lethal than earlier variants but shows no tendency to evade immunity induced by infection or vaccination (Davies et al., 2021; Volz et al., 2021; Abu-Raddad et al., 2021). The B.1.1.7 variant has been detected in 132 countries and rapidly became the dominant variant in Europe and the United States. The B.1.351 variant, first detected in South Africa in May 2020, is not only more transmissible but also capable of reinfecting individuals and of breaking through vaccine protection (Madhi et al., 2021; Johnson & Johnson; Naveca et al., 2021). The B.1.1.28.1 variant is similar to B.1.351 in that both share some important mutations in the spike glycoprotein (E484K, K417N/T, and N501Y). B.1.1.28.1 emerged in late 2020 in Manaus, Brazil (Faria et al., 2021). Similar to the B.1.351 variant, it can cause reinfection because it can bypass immunity developed after infection with other virus variants (Faria et al., 2021; Naveca et al., 2021). It is estimated that B.1.1.28.1 is 40 to 140% more transmissible, more pathogenic, and 10 to 80% more lethal than other variants (Faria et al., 2021). On 7 May 2021, the WHO reclassified the B.1.617.2 variant, first detected in India, as a VOC due to its high transmissibility (WHO). As of August 2021, B.1.617.2 has largely outcompeted B.1.1.7 and became the predominant variant in Europe and the United States. According to the WHO, B.1.617.2 is the most dangerous strain worldwide, and it has attracted considerable attention for its ability to evade infection- and vaccine-mediated protection (Dyer et al., 2021). Variant B.1.1.529 of SARS-CoV-2 was first detected in Botswana in November 2021. It has become the predominant variant in circulation around the world. After the original BA.1 variant, several subvariants of Omicron have evolved: BA.2, BA.3, BA.4, and BA.5, with BA.5 dominating the world as of August 2022.

It is an object of the present invention to provide novel SARS-CoV-2 vaccine candidates.

This object is achieved with a specific polynucleotide encoding a) severe acute respiratory syndrome coronavirus 2 (SARS-CoV-2) spike protein; and optionally b) at least one non-structural SARS-CoV-2 protein selected from the group consisting of non-structural protein 7, non-structural protein 8, non-structural protein 9, non-structural protein 10, non-structural protein 11, non-structural protein 12, an endoribonuclease (also referred to as non-structural protein 15), and a 2'-O-methyltransferase (also referred to as non-structural protein 16). In this context, the polynucleotide comprises or consists of at least one sequence part comprising codon-pair deoptimizations in comparison to the corresponding SARS-CoV-2 genome part. Furthermore, the polynucleotide comprises a furin cleavage site modification resulting in a functional loss of a furin cleavage site being naturally present in the SARS-CoV-2 genome. Thus, the polynucleotide is lacking some genetic information being present in naturally occurring SARS-CoV-2.

The term "furin cleavage site modification", as used herein, refers to a site in the nucleotide sequence of the polynucleotide that corresponds to a site in the SARS-CoV-2 genome encoding for a furin cleavage site but having a modification such that it results in a substantial reduction or loss of (furin) cleavage susceptibility of the encoded cleavage site. Examples of such a modification are demonstrated herein below, e.g., in the examples. The furin cleavage site modification described herein can be any modification that alters the cleavage susceptibility, for example a (partial or full) deletion, an insertion, or a replacement of nucleotides or sequence parts in comparison to the SARS-CoV-2 genome. In some embodiments, the furin cleavage site modification described herein embodies not more than 2, not more than 3, not more than 4, not more than 5, not more than 6, not more than 7, not more than 8, not more than 9, not more than 10, not more than 11, not more than 12, not more than 13, not more than 14, not more than 15, not more than 16, not more than 17, not more than 18, not more than 19, not more than 20, not more than 21, not more than 22, not more than 23, or not more than 24 nucleotide modifications compared to the sequence part of the SARS-CoV-2 encoding the furin cleavage site.

The term "polynucleotide", as used herein, refers to a molecule containing multiple nucleotides (e.g. mRNA, RNA, cRNA, cDNA or DNA). The term typically refers to oligonucleotides greater than 200, preferably greater than 300, preferably greater than 400, preferably greater than 500, preferably greater than 600, preferably greater than 700, preferably greater than 800, preferably greater than 900, preferably greater than 998 nucleotide residues in length. The polynucleotide of the present invention either essentially consists of the nucleic acid sequences described herein or comprises the aforementioned nucleic acid sequences. Thus, it may contain further nucleic acid sequences as well. The term polynucleotide encompasses single stranded as well as double stranded polynucleotides. Moreover, encompassed herein are also modified polynucleotides including chemically modified polynucleotides, artificial modified polynucleotides, or naturally occurring modified polynucleotides such as glycosylated or methylated polynucleotides.

The term "SARS-CoV-2" or "severe acute respiratory syndrome coronavirus 2", as used herein, refers to any variant that is classified as SARS-CoV-2. In some embodiments, the SARS-CoV-2 described herein is at least one SARS-CoV-2 variant selected from the group consisting of Alpha, Beta, Gamma, Delta or Omicron variant. In some embodiments, the SARS-CoV-2 Omicron variant is at least one SARS-CoV-2 Omicron sub-lineage such as BA.1, BA.2, BA.3, BA.4, or BA.5. In some embodiments, the SARS-CoV-2 refers to a SARS-CoV-2 Spike variant comprising at least one mutation selected from the group consisting of del 69-70, RSYLTPGD246-253N, N440K, G446V, L452R, Y453F, S477G/N, E484Q, E484K, F490S, N501Y, N501S, D614G, Q677P/H, P681H, P681R, and A701V. In some embodiments, the SARS-CoV-2 refers to a SARS-CoV-2 variant comprising at least one mutation selected from the group consisting of G142D, G339D, S373P, S375F, K417N, N440K, S477N, T478K, E484A, Q493R, Q498R, N501Y, Y505H, D614G, H655Y, N679K, P681H, N764K, D796Y, Q954H, N969K. In some embodiments, the SARS-CoV-2 refers to a SARS-CoV-2 variant comprising at least one mutation selected from the group consisting of L452R, F486V, and R493Q. Any combination of these mutations is possible and herewith disclosed. Specifically, all mutations listed in either of the precedingly mentioned groups of mutations can be present at the same time within a SARS-CoV-2 variant. Therefore, "SARS-CoV-2 protein" and "SARS-CoV-2 genome" may also be understood as the protein and genome of a SARS-CoV-2 variant, respectively.

The term "codon", as used herein, refers to any group of three consecutive nucleotides in a coding part of a polynucleotide such as a messenger RNA molecule, or coding strand of DNA that specifies a particular amino acid, a starting or stopping signal for translation. Typically, codons are specific for one amino acid, however cases of a codon sharing at least one nucleotide with another codon are known for SARS-CoV-2.

The term "codon pair", as used herein refers to two consecutive codons.

The term "codon-pair deoptimization" (CPD), as used herein, refers to a "reformulation" of codons or an exchange of codons by other codons encoding the same amino acid such that the encoded protein is the same, but suboptimal codon pairs and/or CpG dinucleotides emerge. Methods for codon-pair deoptimizations are known in the art (see, e.g., Coleman et al., 2008, Mueller et al., 2010). In some embodiments, the codon-pair deoptimization described herein comprises increasing the number of underrepresented or suboptimal codon pairs and CpG dinucleotides in recoded genomes. In some embodiments, the codon-pair deoptimization(s) described herein result(s) in increased mRNA decay and/or reduced translation efficiency. In some embodiments, the codon-pair deoptimization(s) described herein result(s) in less protein, less virus, reduced virulence, and/or a live-attenuated virus.

The polynucleotide of the invention may be used in virus production or in the context of a vaccine. As such, the polynucleotide of the invention emits a decreased risk of uncontrolled replication during production, transport, storage, processing and/or administration compared to the wild type sequence.

Typically, this polynucleotide forms part of a live attenuated SARS-CoV-2. In comparison to a wild type virus, a live attenuated virus provokes less and/or less severe or even no symptoms in a host organism after the host organism has been confronted (infected) with the attenuated virus. At the same time, the live attenuated virus induces an immune response of the host to the attenuated virus that is at least partially protective against a wild type virus infection and/or at least one symptom thereof.

In contrast to most of the vaccines under development, the inventors generated attenuated but replicating SARS-CoV-2 vaccine candidates by genetic modification of the SARS-CoV-2 genome via codon pair deoptimization (Coleman et al., 2008). CPD is a virus attenuation strategy which has enabled rapid and highly efficient attenuation of a wide variety of viruses (Broadbent et al., 2016; Coleman et al., 2008; Eschke et al., 2018; Groenke et al., 2020; Khedkar et al., 2018; Kunec and Osterrieder, 2016; Le Nouen et al., 2014; Mueller et al., 2010; Shen et al., 2015; Trimpert et al., 2021a; Trimpert et al., 2021b). CPD rearranges the positions of existing synonymous codons in one or more viral genes, without changing the codon bias or amino acid composition of the encoded protein (Eschke et al., 2018; Groenke et al., 2020; Khedkar et al., 2018; Kunec and Osterrieder, 2016; Osterrieder and Kunec, 2018; Trimpert et al., 2021a; Trimpert et al., 2021b). Naturally underrepresented codons can become overrepresented by CPD. Since the effect of CPD is highly dependent on the genome sequence to be deoptimized, no exact instructions can be given on how the position of codons in the recoded sequence should be changed during deoptimization. However, the person skilled in the art is aware how to identify or estimate codon pairs that can be replaced by naturally underrepresented codon pairs at the target site (e.g. in a target species or a target tissue) at which the polynucleotide is intended to be translated. The inventors provide herein examples which codon pairs to recode to achieve overrepresentation of naturally underrepresented codon pairs and codon-pair deoptimization of the polynucleotide. The skilled person could therefore - at least from the codon pairs at the target site and the means and methods provided herein - arrive at other polynucleotides according to the invention.

A polynucleotide is to be considered as codon-pair deoptimized if at least one codon pair is deoptimized with respect to the corresponding natural sequence. Recoded viruses typically do not produce proteins from the recoded genes as efficiently as their parents, and can show defects in reproductive fitness, which enables the host to control wild-type virus infection by innate and adaptive immune responses (Eschke et al., 2018; Groenke et al., 2020; Khedkar et al., 2018; Kunec and Osterrieder, 2016; Mueller et al., 2010; Trimpert et al., 2021b; Wimmer et al., 2009). The conserved antigenic identity and replicative potential enable recoded attenuated viruses to fully engage the immune system of the host and provoke strong immune responses.

Thus, by the codon-pair deoptimization, the resulting proteins are not altered. Rather, even though the genomic sequence of SARS-CoV-2 is altered, the resulting proteins remain the same. However, typically the efficiency of translation is reduced so that the virus replication is also reduced. This leads to an immune response when the live attenuated SARS-CoV-2 is used as vaccine without the risk of a pathologic virus replication in a patient having received the vaccine. Another possible effect of codon-pair deoptimization is a CpG mediated immune response leading to virus attenuation. The present disclosure is not limited to a specific one of these effects.

The live attenuated SARS-CoV-2 lacking the furin cleavage site (FCS) was severely attenuated but elicited a strong humoral immune response and maintained a similar level of protection in a heterologous SARS-CoV-2 challenge as the live attenuated virus variants with intact FCS and the parental wild-type SARS-CoV-2. Most importantly, however, removal of the FCS completely abolished transmission of vaccine virus between co-housed hamsters. These results indicate that removal of the FCS from live attenuated SARS-CoV-2 is a promising strategy to further increase vaccine safety and prevent vaccine transmission without compromising vaccine efficacy.

It is a very surprising finding that the vaccine efficacy is indeed not compromised by the modification, in particular deletion, of the FCS and the combined attenuating, but independent attenuating mechanisms of codon-pair deoptimization and modification, in particular deletion, of the FCS. In many cases, one can observe "over-attenuating" by combining different attenuating effects, leading to insufficient virus growth or insufficient immune response. Interestingly, such negative over-attenuating effects were not observed for the presently claimed subject matter. In contrast, the modification, in particular deletion, of the FCS results in an increased virus growth in vitro and in an increased genetic stability of the life attenuated SARS-CoV-2. The latter is particularly important with respect to regulatory requirements, since the higher the stability of the life attenuated SARS-CoV-2, the easier it is to obtain a marketing authorization for a vaccine comprising the life attenuated SARS-CoV-2 and the higher is the medical safety of such vaccine.

Unintentional spread of vaccine viruses from vaccinated to unvaccinated individuals is a factor that can complicate the use of transmissible live attenuated vaccines (LAV) (Bull et al., 2018; Layman et al., 2021; Nuismer et al., 2018; Pons-Salort et al., 2016). While self-dissemination is desirable in some scenarios, specifically when herd immunity is sought in wildlife (Smithson et al., 2019), uncontrolled circulation of vaccine viruses potentially increases the likelihood for reversion to virulence (Bull et al., 2018; Layman et al., 2021; Nuismer et al., 2018). Recombination between different vaccine viruses or vaccine and field viruses is especially problematic because it can give rise to recombinants with increased virulence, transmissibility or immune evasion capabilities (Burns et al., 2014; Combelas et al., 2011; Lee et al., 2012; Ming et al., 2020). The rapid evolution of SARS-CoV-2 urges extra caution in the use of LAVs with respect to their potentially irrevocable circulation. Moreover, transmission of attenuated viruses to immunocompromised individuals is a danger that accompanies the use of transmissible virus vaccines (Kamboj and Sepkowitz, 2007).

The inventors already found that sCPD9 confers robust immunity against several SARS-CoV-2 variants in COVID-19 hamster models (Trimpert et al., 2021a; Trimpert et al., 2021b). Most importantly, sCPD9 outperformed intra-muscularly administered adenovirus vector and mRNA vaccines in its ability to induce systemic and mucosal immunity (Nouailles et al., 2022).

The entry of SARS-CoV-2 into host cells is mediated by its major surface protein, the spike protein. The spike protein initiates infection by binding to its cellular receptor, angiotensin-converting enzyme 2 (ACE2), and enables the actual cell entry through fusion between the viral envelope with host cell membranes. To enable infection, the spike protein must be activated by cellular proteases. Activation involves proteolytic cleavage of the spike protein at approximately the midpoint of the protein at the S1/S2 site, resulting in two subunits S1 and S2 that are held together by non-covalent interactions. Cleavage of the spike protein induces conformational changes that enables the S1 subunit to bind to ACE2 via its receptor binding domain and triggers the fusogenic activity of the membrane-anchored S2 subunit.

Unlike other closely related viruses, SARS-CoV-2 contains a unique polybasic cleavage motif (PRRA↓) at the S1/S2 site, termed furin cleavage site (FCS). While several enzymes can cleave the FCS, it is most efficiently cleaved by the cellular transmembrane protease serine 2 (TMPRSS2), a cell surface trypsin-like protease (Hoffmann and Pohlmann, 2021). The TMPRSS2 protease determines the entry pathway of the virus (Koch et al., 2021). When the host cell expresses TMPRSS2, the virus is activated at the cell surface and rapidly enters the cells via cell fusion in a pH-independent manner. In contrast, if TMPRSS2 is absent, the virus is endocytosed and the virus entry is mediated by cathepsin L, an endosomal/lysosomal cysteine protease.

To prevent transmission of the vaccine virus sCPD9, the inventors deleted the FCS from its spike protein. It has been shown in preclinical studies that removal of the FCS renders mutant viruses non-transmissible and strongly attenuated (Johnson et al., 2021; Lau et al., 2020; Peacock et al., 2021; Sasaki et al., 2021a). However, since removal of the FCS can enhance viral attenuation, combining it with a different attenuation mutation may result in an overly attenuated virus with limited ability to induce strong immunity. Therefore, it is important to compare transmissibility and protective efficacy of LAV candidates that lack the FCS with those that have the intact FCS. On the other hand, if removal of the FCS does not compromise the protective effect of the LAV candidates, then removal of the FCS is desirable because it increases the safety of the LAV candidates by introducing a second and independent attenuating mutation into the viral genome.

In addition, aside from eliminating transmission and increasing vaccine safety, removal of the FCS has a potentially important practical advantage for the production of SARS-CoV-2 LAVs. During propagation in cells that do not express TMPRSS2 such as Vero cells, which are commonly used by vaccine manufacturers, SARS-CoV-2 variants lacking a functional FCS become rapidly dominant because they outcompete variants with an intact FCS (Davidson et al., 2020; Klimstra et al., 2020; Lau et al., 2020; Liu et al., 2020; Ogando et al., 2020; Sasaki et al., 2021b; Wong et al., 2021). Consistent with these reports, the inventors found that sCPD9 also rapidly loses its FCS when propagated on cells that do not express TMPRSS2. In contrast, removal of the FCS increases the genetic stability of vaccine viruses during production, and also increases viral titers on TMPRSS2-deficient cell lines.

In an embodiment, the polynucleotide encodes the non-structural protein 7. In an embodiment, the polynucleotide encodes the non-structural protein 8. In an embodiment, the polynucleotide encodes the non-structural protein 9. In an embodiment, the polynucleotide encodes the non-structural protein 10. In an embodiment, the polynucleotide encodes the non-structural protein 11. In an embodiment, the polynucleotide encodes the non-structural protein 12. In an embodiment, the polynucleotide encodes the nsp15, the endonuclease. In an embodiment, the polynucleotide encodes the nsp16, the 2'-O-methyltransferase. In an embodiment, the polynucleotide encodes the spike protein (sometimes also referred to as spike glycoprotein).

In an embodiment, the polynucleotide encodes the spike protein and at least one of the non-structural proteins.

In an embodiment, the polynucleotide encodes at least two of the non-structural proteins. To give an example, the polynucleotide encodes, in an embodiment, the endoribonuclease and the 2'-O-methyltransferase. To give another example, the polynucleotide encodes, in an embodiment, non-structural protein 7, non-structural protein 8, non-structural protein 9, non-structural protein 10, and non-structural protein 11.

In an embodiment, the furin cleavage site modification is a partial or full deletion of a furin cleavage site being naturally present in the SARS-CoV-2 genome.

In an embodiment, the furin cleavage site modification is a loss-of-function mutation of a furin cleavage site being naturally present in the SARS-CoV-2 genome.

In an embodiment, the furin cleavage site modification is an at least partial substitution of a furin cleavage site being naturally present in the SARS-CoV-2 genome.

In an embodiment, the SARS-CoV-2 genome is a genome section extending from position 11,000 to position 27,000 of the genome of SARS-CoV-2. For position numbering and definition of the terms "genome of SARS-CoV-2" and "wild type SARS-CoV-2 ", reference is made to the gene bank accession number MT108784.1 (freely accessible via the website https://www.ncbi.nlm.nih.gov/genbank/) that comprises 29,891 bases or nucleotides. The first of these bases or nucleotides (at the 5' terminus) is positioned at position 1. The last of these bases or nucleotides (at the 3' terminus) is positioned at position 29,891. The skilled person is aware of how to adjust the numbering of the referenced sequence to embodiments, wherein the SARS-CoV-2 genome understood as a sequence from a different SARS-CoV-2 variant. In some embodiments, the polynucleotide of the invention is a codon-pair deoptimized sequence of a sequence comprised in the SARS CoV-2 genome section from position 11,000 to position 24,000. In an embodiment, the genome section extends from position 11,500 to position 26,000, in particular from position 11,900 to position 25,500, in particular from position 11,950 to position 25,350, in particular from position 12,000 to position 24,000. In an embodiment, the genome section extends from position 11,950 to position 14,400. In an embodiment, the genome section extends from position 11,900 to position 13,500. In an embodiment, the genome section extends from position 13,900 to position 14,400. In an embodiment, the genome section extends from position 20,300 to position 21,600. In an embodiment, the genome section extends from position 24,300 to position 25,400. These embodiments can be combined in any desired way.

In an embodiment, the at least one sequence part comprising codon-pair deoptimizations has a length lying in a range of from 750 nucleotides to 2500 nucleotides, in particular of from 800 nucleotides to 2400 nucleotides, in particular of from 900 nucleotides to 2300 nucleotides, in particular of from 999 nucleotides to 2200 nucleotides, in particular of from 1000 nucleotides to 2100 nucleotides, in particular of from 1100 nucleotides to 2000 nucleotides, in particular of from 1146 nucleotides to 1900 nucleotides, in particular of from 1200 nucleotides to 1836 nucleotides, in particular of from 1300 nucleotides to 1800 nucleotides, in particular of from 1400 nucleotides to 1700 nucleotides, in particular of from 1500 nucleotides to 1600 nucleotides.

In an embodiment, between 15 % and 40 %, in particular between 20 % and 35 %, in particular between 25 % and 30 % of the nucleotides of the at least one sequence part comprising codon-pair deoptimizations are different from the nucleotides of a corresponding (wild type) SARS-CoV-2 genome. Such a wild-type SARS-CoV-2 genome is the genomic sequence of a non-artificially modified virus variant or lineage, such as lineages B.1.1.7 (Alpha), B.1.351 (Beta), B.1.1.28.1 (Gamma), B.1.617.2 (Delta), or B.1.159.1 (Omicron), including any sub-variants such as Omicron sub-variants BA.1, BA.2, BA.3, BA.4, BA.5. It can also be denoted as authentic SARS-CoV-2 genome or authentic SARS-CoV-2 genomic sequence.

In an embodiment, between 200 and 500 nucleotides, in particular between 250 and 450 nucleotides, in particular between 300 and 400 nucleotides of the at least one sequence part comprising codon-pair deoptimizations are different from the (in particular identically positioned) nucleotides of a corresponding SARS-CoV-2 genome.

In an embodiment, between 40 % and 70 %, in particular between 45 % and 65 %, in particular between 50 % and 60 %, in particular between 55 % and 62 % of the codons of the at least one sequence part comprising codon-pair deoptimizations are different from the respective codons of a corresponding SARS-CoV-2 genome.

In an embodiment, between 150 and 400 codons, in particular between 200 and 350 codons, in particular between 250 and 300 codons of the at least one sequence part comprising codon-pair deoptimizations are different from the (in particular identically positioned) codons of a corresponding SARS-CoV-2 genome.

In an embodiment, the at least one sequence part comprising codon-pair deoptimizations comprises a first deoptimized sequence part and a second deoptimized sequence part. Both deoptimized sequence parts are separated from each other by a non-deoptimized sequence section comprising at least 300 nucleotides, e.g., 300 to 1000 nucleotides, in particular 400 to 900 nucleotides, in particular 500 to 800 nucleotides, in particular 600 to 700 nucleotides. By conserving a specific part of the RNA sequence and by deoptimizing flanking parts upstream and downstream of the conserved RNA sequence, a particularly high efficacy in attenuating the SARS-CoV-2 is achieved while maintaining its ability to replicate.

In an embodiment, the first deoptimized sequence part has a length lying in a range of from 1300 nucleotides to 1600 nucleotides, in particular of from 1400 nucleotides to 1500 nucleotides, in particular of from 1450 nucleotides to 1490 nucleotides. At the same time, the second deoptimized sequence part has a length lying in a range of from 100 nucleotides to 400 nucleotides, in particular of from 200 nucleotides to 300 nucleotides, in particular of from 350 nucleotides to 400 nucleotides. The length of the first deoptimized sequence part and of the second deoptimized sequence part is chosen such that other applicable restrictions (such as an overall length of the at least one sequence part comprising codon-pair deoptimizations of not more than 2000 nucleotides) are fulfilled, if desired. If the length of the at least one sequence part comprising codon-pair deoptimizations shall not exceed 2000 nucleotides, it is immediately apparent that only the lower threshold of 1300 nucleotides can be combined with the upper threshold of 400 nucleotides for the first and second deoptimized sequence parts to fulfil the restriction of the maximum length of the at least one sequence part comprising codon-pair deoptimizations, considering that the first deoptimized sequence part and the second deoptimized sequence part are separated by at least 300 nucleotides of the authentic SARS-CoV-2 genome. At the same time, the upper threshold of 1600 nucleotides for the first deoptimized sequence part can be combined with the lower threshold of 100 nucleotides for the second deoptimized sequence part to fulfil a maximum length of 2000 nucleotides, considering the intermediate 300 non-recoded nucleotides.

In an embodiment, the first deoptimized sequence part is at least 95 %, in particular at least 96 %, in particular at least 97 %, in particular at least 98 %, in particular at least 99 %, in particular 100 % identical to SEQ ID NO. 2. At the same time, the second deoptimized sequence part is at least 95 %, in particular at least 96 %, in particular at least 97 %, in particular at least 98 %, in particular at least 99 %, in particular 100 % identical to SEQ ID NO. 4.

In an embodiment, the polynucleotide is at least 95 %, in particular at least 96 %, in particular at least 97 %, in particular at least 98 %, in particular at least 99 %, in particular 100 % identical to SEQ ID NO. 6.

In an embodiment, the polynucleotide is at least 95 %, in particular at least 96 %, in particular at least 97 %, in particular at least 98 %, in particular at least 99 %, in particular 100 % identical to SEQ ID NO. 8.

In an embodiment, the polynucleotide is at least 95 %, in particular at least 96 %, in particular at least 97 %, in particular at least 98 %, in particular at least 99 %, in particular 100 % identical to SEQ ID NO. 10.

In an embodiment, the polynucleotide is at least 95 %, in particular at least 96 %, in particular at least 97 %, in particular at least 98 %, in particular at least 99 %, in particular 100 % identical to SEQ ID NO. 15.

In an embodiment, the polynucleotide is at least 95 %, in particular at least 96 %, in particular at least 97 %, in particular at least 98 %, in particular at least 99 %, in particular 100 % identical to SEQ ID NO. 16.

In an embodiment, the polynucleotide is at least 95 %, in particular at least 96 %, in particular at least 97 %, in particular at least 98 %, in particular at least 99 %, in particular 100 % identical to SEQ ID NO. 17.

In an embodiment, the furin cleavage site modification comprises a deletion of the nucleotides encoding an amino acid sequence XRRA (i.e., a furin cleavage site), wherein X denotes P, R or H. The following Table 1 lists specific embodiments of the presently claimed subject matter relating to different amino acid sequences missing in the expressed protein due to the deletion of the furin cleavage site. It should be noted that it is less relevant which nucleotides are excised from the SARS-CoV-2 genome by the deletion of the furin cleavage site, as long as the furin cleavage site is no longer present in the resulting protein.

**Table 1: Embodiments of missing amino acids due to the deletion of the furin cleavage site.**

| **Sequence name** | **SEQ ID NO.** | **Missing amino acids due to the deletion of the furin cleavage site** |
|---|---|---|
| DelFCS variant 1 | 21 | PRRA |
| DelFCS variant 2 | 22 | PRRAR |
| DelFCS variant 3 | 23 | PRRARS |
| DelFCS variant 4 | 24 | PRRARSV |
| DelFCS variant 5 | 25 | SPRRA |
| DelFCS variant 6 | 26 | SPRRAR |
| DelFCS variant 7 | 27 | SPRRARS |
| DelFCS variant 8 | 28 | SPRRARSV |
| DelFCS variant 9 | 29 | NSPRRA |
| DelFCS variant 10 | 30 | NSPRRAR |
| DelFCS variant 11 | 31 | NSPRRARS |
| DelFCS variant 12 | 32 | NSPRRARSV |
| DelFCS variant 13 | 33 | RRRA |
| DelFCS variant 14 | 34 | RRRAR |
| DelFCS variant 15 | 35 | RRRARS |
| DelFCS variant 16 | 36 | RRRARSV |
| DelFCS variant 17 | 37 | SRRRA |
| DelFCS variant 18 | 38 | SRRRAR |
| DelFCS variant 19 | 39 | SRRRARS |
| DelFCS variant 20 | 40 | SRRRARSV |
| DelFCS variant 21 | 41 | NSRRRA |
| DelFCS variant 22 | 42 | NSRRRAR |
| DelFCS variant 23 | 43 | NSRRRARS |
| DelFCS variant 24 | 44 | NSRRRARSV |
| DelFCS variant 25 | 45 | HRRA |
| DelFCS variant 26 | 46 | HRRAR |
| DelFCS variant 27 | 47 | HRRARS |
| DelFCS variant 28 | 48 | HRRARSV |
| DelFCS variant 29 | 49 | SHRRA |
| DelFCS variant 30 | 50 | SHRRAR |
| DelFCS variant 31 | 51 | SHRRARS |
| DelFCS variant 32 | 52 | SHRRARSV |
| DelFCS variant 33 | 53 | NSHRRA |
| DelFCS variant 34 | 54 | NSHRRAR |
| DelFCS variant 35 | 55 | NSHRRARS |
| DelFCS variant 36 | 56 | NSHRRARSV |

In an embodiment, the furin cleavage site modification, in particular deletion, effects that an expression of the polynucleotide results in a protein, in particular a spike protein, in which at least or exactly 5, in particular at least or exactly 6, in particular at least or exactly 7, in particular at least or exactly 8, in particular at least or exactly 9, in particular at least or exactly 10 consecutive amino acids of the naturally expressed protein are replaced by a single amino acid. This can be achieved by an off-reading-frame deletion of nucleotides. By such an off-reading-frame deletion, a single nucleotide of a first triplet is combined with two nucleotides of a second triplet to form a novel triplet that is not present in the natural SARS-CoV-2 genome at this genome position.

In an embodiment, the single amino acid that replaces the naturally expressed consecutive amino acids is an isoleucine.

In an embodiment, the furin cleavage site modification consists of or comprises a deletion of nucleic acid sequence as defined by SEQ ID NO. 18 or a nucleic acid sequence having at least 95 % sequence identity to SEQ ID NO. 18.

In an aspect, the present invention relates to a live attenuated SARS-CoV-2. This live attenuated SARS-CoV-2 comprises a partially recoded genomic RNA sequence, i.e., a partially recoded genomic viral sequence. The partially recoded genomic RNA sequence is a codon-pair deoptimized sequence coding for a spike protein and/or a specific non-structural protein (nsp). The non-structural protein is chosen from the group consisting of non-structural protein 7, non-structural protein 8, non-structural protein 9, non-structural protein 10, non-structural protein 11 (a small, only 13 amino acids long protein), non-structural protein 12 (also referred to as RNA-dependent RNA polymerase), non-structural protein 15 (an endoribonuclease), and non-structural protein 16 (a 2'-O-methyltransferase) of the live attenuated SARS-CoV-2. The live attenuated SARS-CoV-2 further comprises a furin cleavage site modification resulting in a functional loss of a furin cleavage site. This furin cleavage site is naturally present in the SARS-CoV-2 genome. As a result, the spike protein of the live attenuated SARS-CoV-2 does not comprise a functional furin cleavage site, whereas non-engineered SARS-CoV-2 does comprise such furin cleavage site.

In an embodiment, the partially recoded genomic RNA sequence codes for the non-structural protein 12. In an embodiment, the partially recoded genomic sequence codes for the spike protein (sometimes also referred to as spike glycoprotein).

In an embodiment, the partially recoded genomic RNA sequence comprises at least two of the non-structural proteins. To give an example, the partially recoded genomic RNA sequence codes, in an embodiment, for the endoribonuclease and the 2'-O-methyltransferase. To give another example, the partially recoded genomic RNA sequence codes, in an embodiment, for non-structural protein 7, non-structural protein 8, non-structural protein 9, non-structural protein 10, and non-structural protein 11.

In an embodiment, the partially recoded genomic RNA sequence lies in a genome section extending from position 11,000 to position 27,000 of the genome of the live attenuated SARS-CoV-2. According to gene bank accession number MT108784.1, the genome of the wild type SARS-CoV-2 comprises 29,891 bases or nucleotides. The genome of the live attenuated SARS-CoV-2 has essentially a similar length. A difference is the length of a polyA tail at the 3' terminus that was determined by sequencing to be eight adenine nucleotides longer than in case of the wild type SARS-CoV-2. It should be noted that some uncertainty remains by determining the length of a polyA tail by sequencing. Consequently, it is possible that the polyA tail in the wild type sequence is longer or shorter than indicated in the sequence according to gene bank accession number MT108784.1. Likewise, it is possible that the polyA tail in the live attenuated SARS-CoV-2 sequence is longer or shorter than presently determined. A further difference is that the live attenuated SARS-CoV-2 lacks at least 12 nucleotides that encode the furin cleavage site in the wild type SARS-CoV-2.

The first of the 29,891 bases or nucleotides of the genome of the wild type SARS-CoV-2 (at the 5' terminus) is positioned at position 1. The last of these bases or nucleotides (at the 3' terminus) is positioned at position 29,891. In an embodiment, the genome section extends from position 11,500 to position 26,000, in particular from position 11,900 to position 25,500, in particular from position 11,950 to position 25,350, in particular from position 12,000 to position 24,000. In an embodiment, the genome section extends from position 11,950 to position 14,400. In an embodiment, the genome section extends from position 11,900 to position 13,500. In an embodiment, the genome section extends from position 13,900 to position 14,400. In an embodiment, the genome section extends from position 20,300 to position 21,600. In an embodiment, the genome section extends from position 24,300 to position 25,400. These embodiments can be combined in any desired way.

In an embodiment, the partially recoded genomic RNA sequence has a length lying in a range of from 750 nucleotides to 2500 nucleotides, in particular of from 800 nucleotides to 2400 nucleotides, in particular of from 900 nucleotides to 2300 nucleotides, in particular of from 999 nucleotides to 2200 nucleotides, in particular of from 1000 nucleotides to 2100 nucleotides, in particular of from 1100 nucleotides to 2000 nucleotides, in particular of from 1146 nucleotides to 1900 nucleotides, in particular of from 1200 nucleotides to 1836 nucleotides, in particular of from 1300 nucleotides to 1800 nucleotides, in particular of from 1400 nucleotides to 1700 nucleotides, in particular of from 1500 nucleotides to 1600 nucleotides.

In an embodiment, between 15 % and 40 %, in particular between 20 % and 35 %, in particular between 25 % and 30 % of the nucleotides of the partially recoded genomic RNA sequence are different from the nucleotides of a corresponding wild-type genomic RNA sequence. Such a wild-type genomic RNA sequence is the genomic viral sequence of a non-artificially modified virus variant or lineage, such as lineages B.1.1.7 (Alpha), B.1.351 (Beta), B.1.1.28.1 (Gamma), B.1.617.2 (Delta), or B.1.159.1 (Omicron). It can also be denoted as authentic SARS-CoV-2 genomic RNA sequence.

In an embodiment, between 200 and 500 nucleotides, in particular between 250 and 450 nucleotides, in particular between 300 and 400 nucleotides of the partially recoded genomic RNA sequence are different from the identically positioned nucleotides of a corresponding wild-type virus genomic RNA sequence.

In an embodiment, between 40 % and 70 %, in particular between 45 % and 65 %, in particular between 50 % and 60 %, in particular between 55 % and 62 % of the codons (i.e., three nucleotides in each case that code for a specific amino acid) of the partially recoded genomic RNA sequence are different from the respective codons of a corresponding wild-type virus genomic RNA sequence.

In an embodiment, between 150 and 400 codons, in particular between 200 and 350 codons, in particular between 250 and 300 codons of the partially recoded genomic RNA sequence are different from the identically positioned codons of a corresponding wild-type virus genomic RNA sequence.

In an embodiment, the partially recoded genomic RNA sequence comprises a first recoded part and a second recoded part. Both recoded parts are separated from each other by a non-recoded genome section comprising at least 300 nucleotides, e.g., 300 to 1000 nucleotides, in particular 400 to 900 nucleotides, in particular 500 to 800 nucleotides, in particular 600 to 700 nucleotides. By conserving a specific part of the RNA sequence and by recoding flanking parts upstream and downstream of the conserved RNA sequence, a particularly high efficacy in attenuating the SARS-CoV-2 while maintaining its general viability is achieved.

In an embodiment, the first recoded part has a length lying in a range of from 1300 nucleotides to 1600 nucleotides, in particular of from 1400 nucleotides to 1500 nucleotides, in particular of from 1450 nucleotides to 1490 nucleotides. At the same time, the second recoded part has a length lying in a range of from 100 nucleotides to 400 nucleotides, in particular of from 200 nucleotides to 300 nucleotides, in particular of from 350 nucleotides to 400 nucleotides. The length of the first recoded part and of the second recoded part is chosen such that other applicable restrictions (such as an overall length of the recoded genomic RNA sequence of not more than 2000 nucleotides) are fulfilled, if desired. If the length of the partially recoded genomic RNA sequence shall not exceed 2000 nucleotides, it is immediately apparent that only the lower threshold of 1300 nucleotides can be combined with the upper threshold of 400 nucleotides for the first and second recoded parts to fulfil the restriction of the maximum length of the partially recoded genomic RNA sequence, considering that the first recoded part and the second recoded part are separated by at least 300 nucleotides of the authentic SARS-CoV-2 genomic sequence. At the same time, the upper threshold of 1600 nucleotides for the first recoded part can be combined with the lower threshold of 100 nucleotides for the second recoded part to fulfil a maximum length of 2000 nucleotides, considering the intermediate 300 non-recoded nucleotides.

In an embodiment, the first recoded part is at least 95 %, in particular at least 96 %, in particular at least 97 %, in particular at least 98 %, in particular at least 99 %, in particular 100 % identical to SEQ ID NO. 2. At the same time, the second recoded part is at least 95 %, in particular at least 96 %, in particular at least 97 %, in particular at least 98 %, in particular at least 99 %, in particular 100 % identical to SEQ ID NO. 4.

In an embodiment, the partially recoded genomic RNA sequence is at least 95 %, in particular at least 96 %, in particular at least 97 %, in particular at least 98 %, in particular at least 99 %, in particular 100 % identical to SEQ ID NO. 6.

In an embodiment, the partially recoded genomic RNA sequence is at least 95 %, in particular at least 96 %, in particular at least 97 %, in particular at least 98 %, in particular at least 99 %, in particular 100 % identical to SEQ ID NO. 8.

In an embodiment, the partially recoded genomic RNA sequence is at least 95 %, in particular at least 96 %, in particular at least 97 %, in particular at least 98 %, in particular at least 99 %, in particular 100 % identical to SEQ ID NO. 10.

The phrase "being % identical" or "having percent (%) sequence identity" with respect to a reference sequence is defined as the percentage of nucleotides or amino acid residues in a candidate sequence that are identical with the nucleotides or amino acid residues in the reference sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for aligning sequences, including any algorithms needed to achieve maximal alignment over the full length of the sequences being compared.

The skilled person is aware that sequences as exemplified herein can be altered to a certain percentage without or without substantially altering biologic functions such as attenuation or the encoded protein. Therefore, the skilled person using means and methods described herein can amend codon pairs according to the teaching of the invention. E.g., codon pairs can be replaced with synonymous versions that are similarly attenuating and/or naturally underrepresented as the deoptimized codon pairs of the sequences described herein. Thus, a similar degree of deoptimization may be achieved by replacing codon pairs in the position ranges described herein.

The invention is based at least in part on the finding that codon deoptimizations in the position ranges described herein are particularly useful for attenuating the SARS-CoV-2 virus while sufficiently maintaining immunogenicity and the ability of the virus to replicate. It is further based on the finding that a furin cleavage site modification, which results in a functional loss of a furin cleavage site being naturally present in the SARS-CoV-2 genome, further attenuates the SARS-CoV-2, while fully maintaining its immunogenicity.

The individual recoded sequences and their position in the SARS-CoV-2 genome are summarized in the following Table 2.

**Table 2: Summary of recoded RNA sequences.**

| **Sequence name** | **SEQ ID NO.** | **Description** | **Recoded protein** | **Length (nt)** | **Length (aa)** | **Position in SARS-CoV-2 genome (NC_045512.2)** | |
|---|---|---|---|---|---|---|---|
| | | | | | | **Start** | **End** |
| WT6A | 1 | first part of original sequence, to be replaced by first part of CPD sequence | pp1a/pp1ab - nsp7, nsp8, nsp9, nsp10, nsp11 and nsp12 | 1.482 | 494 | 11.969 | 13.450 |
| CPD6A | 2 | first part of CPD sequence | Ppla/pplab - nsp7, nsp8, nsp9,nsp10, nsp11 and nsp12 | 1.482 | 494 | 11.969 | 13.450 |
| WT6B | 3 | second part of original sequence, to be replaced by second part of CPD sequence | pplab - nsp12 | 354 | 118 | 13.954 | 14.307 |
| CPD6B | 4 | second part of CPD sequence | pplab - nsp12 | 354 | 118 | 13.954 | 14.307 |
| WT6 | 5 | original, to be replaced by CPD sequence | pp1a/pp1ab - nsp7, nsp8, nsp9, nsp10, nsp11 and nsp12 | 1482 +503 +354 =2339 | 494 +118 =612 | 11.969 | 14.307 |
| CPD6 | 6 | CPD sequence | pp1a/pp1ab - nsp7, nsp8, nsp9, nsp10, nsp11 and nsp12 | 1482 +503 +354 =2339 | 494 +118 =612 | 11.969 | 14.307 |
| sWT9 | 7 | original, to be replaced by CPD sequence | pplab - endoribonuclease and 2'-O-methyltransferase | 1.146 | 382 | 20.359 | 21.504 |
| sCPD9 | 8 | CPD sequence | pplab - endoribonuclease and 2'-O-methyltransferase | 1.146 | 382 | 20.359 | 21.504 |
| sWT10 | 9 | original, to be replaced by CPD sequence | Spike | 999 | 333 | 24.335 | 25.333 |
| sCPD10 | 10 | CPD sequence | Spike | 999 | 333 | 24.335 | 25.333 |

| | | | | | | | |
|---|---|---|---|---|---|---|---|
| pp = polyprotein nsp = non-structural protein | | | | | | | |

It should be noted that WT6A/CPD6A encodes only nine nucleotides (three amino acids) of nsp12 (RNA-dependent RNA polymerase, RdRp). The majority of this protein is encoded by WT6B/CPD6B.

The middle part of fragment WT6 was not recoded in fragment CPD6, because it contains a conserved regulatory RNA sequence that is essential for virus replication. Fragment WT6 encodes a -1 ribosomal frameshifting element, a so-called RNA pseudoknot structure. This structure promotes a ribosomal frameshifting, a process during which the reading frame of translation is changed at the junction between open reading frames (ORFs) 1a and 1b. During this process, a single nucleotide of the slippery sequence that is located downstream from the RNA pseudoknot structure is read twice by the translating ribosome, and the reading frame is shifted by -1 nucleotide (the ribosome slips one nucleotide backwards at the slippery sequence). Often, translation of ORF1a terminates at the stop codon of ORF1a. However, when -1 ribosomal frameshifting occurs, translation of ORF1a continues directly to ORF1b and polyprotein (pp) 1ab is produced. Thus, the CPD6A sequence is translated into both polyproteins 1a and 1ab; but the CPD6B sequence is translated only into polyprotein 1ab.

The differences between the CPD sequences and the underlying wild type sequences are summarized in the following Table 3.

**Table 3: Summary of difference between CPD and wild type sequences.**

| **Sequence name** | **SEQ ID NO.** | **Preserved nucleotides with respect to wild type** | **Altered nucleotides with respect to wild type** | **Different nucleotides with respect to wild type** | **Preserved codons with respect to wild type** | **Altered codons with respect to wild type** | **Different codons with respect to wild type** |
|---|---|---|---|---|---|---|---|
| CPD6A | 2 | 1.144 | 338 | 22.8% | 206 | 288 | 58.3% |
| CPD6B | 4 | 270 | 84 | 23.7% | 48 | 70 | 59.3% |
| sCPD9 | 8 | 907 | 239 | 20.9% | 177 | 205 | 53.7% |
| sCPD10 | 10 | 758 | 241 | 24.1% | 132 | 201 | 60.4% |

It should be noted that the primary structure of the proteins encoded by the CPD RNA sequences is identical to the primary structure of the wild-type (original) RNA sequences. As explained above, CPD does not alter the primary structure of the resulting protein. This is summarized in Table 4.

**Table 4: Summary of protein sequences.**

| **RNA Sequence name** | **RNA SEQ ID NO.** | **Recoded protein** | **Protein SEQ ID NO.** |
|---|---|---|---|
| WT6A | 1 | ORF1a/ORF1ab - nsp7, nsp8, nsp9, nsp10, nsp11 and nsp12 | 11 |
| CPD6A | 2 | ORF1a/ORF1ab - nsp7, nsp8, nsp9 and nsp10, nsp11 and nsp12 | |
| WT6B | 3 | ORFlab - nsp12 | 12 |
| CPD6B | 4 | ORF1ab-nsp12 | |
| sWT9 | 7 | ORFlab - endoribonuclease and 2'-O-methyltransferase | 13 |
| sCPD9 | 8 | ORFlab - endoribonuclease and 2'-O-methyltransferase | |
| sWT10 | 9 | Spike | 14 |
| sCPD10 | 10 | Spike | |

By recoding sequence parts CPD6A, CPD6B, sCPD9, and sCPD10, different fragments of the SARS-CoV-2 genome were generated that form part of a live attenuated SARS-CoV-2 in an embodiment. These fragments are listed in the following Table 5.

**Table 5: Genome fragments of SARS-CoV-2 comprising recoded sequence parts.**

| **Genome fragment name** | **Length (nucleotides)** | **SEQ ID NO.** | **Accession number** | **Website providing access to sequence** |
|---|---|---|---|---|
| Fragment 6 comprising CPD6A and CPD6B | 2.943 | 15 | MZ064535 | http://getentry.ddbj.nig.ac.jp/getentry /na/MZ064535?filetype=html |
| Fragment 9 comprising sCPD9 | 3.178 | 16 | MZ064545 | http://getentry.ddbj.nig.ac.jp/getentry /na/MZ064545?filetype=html |
| Fragment 10 comprising sCPD10 | 2.966 | 17 | MZ064546 | http://getentry.ddbj.nig.ac.jp/getentry /na/MZ064546?filetype=html |

In an aspect, the present invention relates to a live attenuated severe acute respiratory syndrome coronavirus 2 (SARS-CoV-2) comprising the polynucleotide according the above explanations.

In an embodiment, the live attenuated SARS-CoV-2 has a nucleic acid sequence being at least 98 %, in particular at least 99 %, in particular 100 % identical to SEQ ID NO. 19. Besides a codon-pair deoptimized region, this live attenuated SARS-CoV-2 comprises a 24-nucleotide deletion of a furin cleavage site in the spike protein gene, leading to a replacement of the amino acid sequence NSPRRARSV (SEQ ID NO. 32) comprising a furin cleavage site by the amino acid isoleucine in the spike glycoprotein.

In an embodiment, the SARS-CoV-2 has a nucleic acid sequence being at least 98 %, in particular at least 99 %, in particular 100 % identical to SEQ ID NO. 20. Besides a codon-pair deoptimized region, also this live attenuated SARS-CoV-2 comprises a 24-nucleotide deletion of a furin cleavage site in the spike protein gene, leading to a replacement of the amino acid sequence NSPRRARSV (SEQ ID NO. 32) comprising a furin cleavage site in the spike protein with the amino acid isoleucine.

In an aspect, the present invention relates to a pharmaceutical composition comprising the live attenuated SARS-CoV-2 according to any of the preceding explanations. Such a pharmaceutical composition can further comprise auxiliary substances like adjuvants, e.g., for enhancing an immune response of a patient. Appropriate adjuvants are potassium alum; aluminium hydroxide; aluminium phosphate; calcium phosphate hydroxide; aluminum hydroxyphosphate sulfate; paraffin oil; propolis; killed bacteria of the species *Bordetella pertussis* or *Mycobacterium bovis*; plant saponins from *Quillaja,* soybean, and/or *Polygala senega;* cytokines IL-1, IL-2, and/or IL-12; as well as Freund's complete adjuvant.

In an aspect, the present invention relates to the further medical use of such a pharmaceutical composition as a vaccine.

In an aspect, the present invention relates to a method for preparing a vaccine from such a pharmaceutical composition.

In an aspect, the present invention relates to a method of vaccinating a human or animal patient in need thereof. The animal patient is in particular a non-human mammal such as a rodents, canines, felines, or mustelids. This method comprises the step of administering a pharmaceutical composition according to the preceding explanations to the patient.

In an embodiment, the administration is performed by an intranasal administration, an oral administration, a parenteral administration such as a subcutaneous injection, an intramuscular injection, an intravenous injection, an intraperitoneal injection, an intravenous infusion, or an intraperitoneal infusion. An intranasal or oral administration is particularly appropriate. By these routes of administration, the live attenuated SARS-CoV-2 is presented to the body of the patient to be vaccinated in the same or a similar way as in case of a natural exposure to the virus.

In an embodiment, the vaccination is performed by administering the pharmaceutical composition in a dose comprising between 1*10³ and 1*10⁸ focus-forming units (FFU), in particular between 1*10⁴ and 1*10⁷ FFU, in particular between 1*10⁵ and 1*10⁶ FFU, of the live attenuated SARS-CoV-2. The dose is chosen such that the pharmaceutical composition is well tolerated by the patient but evokes an immune response that gives protection to the patient against an infection or a severe course of an infection with SARS-CoV-2. In embodiment, the dose is one of the lowest protective dose and the highest tolerable dose or lies between the lowest protective dose and the highest tolerable dose.

Various factors can influence the dose used for a particular application. For example, the frequency of administration, duration of treatment, preventive or therapeutic purpose, the use of multiple treatment agents, route of administration, previous therapy, the patient's clinical history, the discretion of the attending physician and severity of the disease, disorder and/or condition may influence the required dose to be administered.

As with the dose, various factors can influence the actual frequency of administration used for a particular application. For example, the dose, duration of treatment, use of multiple treatment agents, route of administration, and severity of the disease, disorder and/or condition may require an increase or decrease in administration frequency.

In some cases, an effective duration for administering the pharmaceutical composition of the invention (and any additional therapeutic agent) can be any duration that reduces the severity, or occurrence, of symptoms of the disease, disorder and/or condition to be treated without producing significant toxicity to the subject. Multiple factors can influence the actual effective duration used for a particular treatment. For example, an effective duration can vary with the frequency of administration, effective amount, use of multiple treatment agents, route of administration, and severity of the disease, disorder and/or condition being treated.

In an embodiment, the pharmaceutical preparation is administered to the patient at least two times, wherein the second administration is separated from the first administration by a first time period. In this context, the first time period lies in a range of from 2 weeks to 36 months, in particular of from 3 weeks to 30 months, in particular of from 4 weeks to 24 months, in particular of from 5 weeks to 21 months, in particular of from 6 weeks to 18 months, in particular of from 7 weeks to 15 months, in particular of from 8 weeks to 12 months, in particular of from 9 weeks to 10 months, in particular of from 10 weeks to 8 months, in particular of from 12 weeks to 6 months, in particular of from 13 weeks to 4 months.

In an embodiment, the pharmaceutical preparation is administered to the patient temporally offset to administering a different vaccine (such as, e.g., a vector-based vaccine, an mRNA-based vaccine, a protein-based vaccine) to the patient, i.e., after or before vaccinating the patient with the different vaccine. In this context, the administration of the pharmaceutical composition is offset to the administration of the different vaccine by a second time period. In this context, the second time period lies in a range of from 2 weeks to 36 months, in particular of from 3 weeks to 30 months, in particular of from 4 weeks to 24 months, in particular of from 5 weeks to 21 months, in particular of from 6 weeks to 18 months, in particular of from 7 weeks to 15 months, in particular of from 8 weeks to 12 months, in particular of from 9 weeks to 10 months, in particular of from 10 weeks to 8 months, in particular of from 12 weeks to 6 months, in particular of from 13 weeks to 4 months.

In an aspect, the present invention relates to a vector comprising the polynucleotide according to the above explanations.

The term "vector", as used herein, refers to a nucleic acid molecule, capable of transferring or transporting itself and/or another nucleic acid molecule into a cell. The transferred nucleic acid is generally linked to, i.e., inserted into, the vector nucleic acid molecule. A vector may include sequences that direct autonomous replication in a cell, or may include sequences sufficient to allow integration into host cell DNA. In some embodiments, the vector described herein is a vector selected from the group of plasmids (e.g., DNA plasmids or RNA plasmids), shuttle vectors, transposons, cosmids, artificial chromosomes (e.g. bacterial, yeast, human), and viral vectors.

In some embodiments, the vector described herein is used in combination with at least one transfection enhancer, e.g., a transfection enhancer selected from the group of oligonucleotides, lipoplexes, polymersomes, polyplexes, dendrimers, inorganic nanoparticles and cell-penetrating peptides.

In an aspect, the present invention relates to a host cell comprising the polynucleotide according to the above explanations.

The term "host cell", as used herein, refers to a cell into which exogenous nucleic acid has been introduced, including the progeny of such a cell. Host cells include "transformants" and "transformed cells", which include the primary transformed cell and progeny derived therefrom without regard to the number of passages. Progeny may not be completely identical in nucleic acid content to a parent cell but may contain mutations. Mutant progeny that have the same function or biological activity as screened or selected for in the originally transformed cell are included herein.

In an embodiment, the host cell described herein comprises at least one cell type selected from the group of Chinese hamster ovary (CHO), Vero, Vero E6, Vero TMPRSS, MRC 5, Per.C6, PMK, WI-38, baby hamster kidney fibroblasts (BHK cells).

In an aspect, the present invention relates to a method for the production of a virus. This method comprises the steps of a) culturing a host cell according to the preceding paragraph; and b) isolating a virus, wherein the virus is a live attenuated SARS-CoV-2.

All embodiments of the polynucleotide can be combined in any desired way and can be transferred either individually or in any arbitrary combination to the live attenuated SARS-CoV-2, to the pharmaceutical composition, its use, to the method of vaccinating the patient, to the vector, to the host cell, and to the method of producing a virus. All embodiments of the live attenuated SARS-CoV-2 can be combined in any desired way and can be transferred either individually or in any arbitrary combination to the polynucleotide, to the pharmaceutical composition, its use, to the method of vaccinating the patient, to the vector, to the host cell, and to the method of producing a virus. Likewise, all embodiments of the pharmaceutical composition can be combined in any desired way and can be transferred either individually or in any arbitrary combination to the polynucleotide, to the live attenuated SARS-CoV-2, the use of the pharmaceutical composition, to the method of vaccinating a patient, to the vector, to the host cell, and to the method of producing a virus. All embodiments of the use of the pharmaceutical preparation can be combined in any desired way and can be transferred either individually or in any arbitrary combination to the polynucleotide, to the live attenuated SARS-CoV-2, to the pharmaceutical preparation, to the method of vaccinating a patient, to the vector, to the host cell, and to the method of producing a virus. Finally, all embodiments of the method of vaccinating a patient can be combined in any desired way and can be transferred either individually or in any arbitrary combination to the polynucleotide, to the live attenuated SARS-CoV-2, to the pharmaceutical preparation, to the use of the pharmaceutical preparation, to the vector, to the host cell, and to the method of producing a virus.

Further details of aspects of the present invention will be explained in the following making reference to exemplary embodiments and accompanying Figures. In the Figures:
- Figures 1A to 1D: schematically depict the native structure and exemplary recoding of the SARS-CoV-2 genome;
- Figure 2: shows the growth kinetics of sCPD9-ΔFCS and sCPD9 SARS-CoV-2 in Vero E6 cells;
- Figures 3A to 3E: illustrate virological and histopathological findings in contact hamsters;
- Figures 4A and 4B: illustrate clinical parameters of infected Syrian hamsters; and
- Figures 5A to 5E: illustrate clinical, virological, pathological and serological results found in hamsters after challenge-infection with SARS-CoV-2 Delta variant.

### EXEMPLARY EMBODIMENT

The inventors generated a series of recoded SARS-CoV-2 mutants, characterized them in cultured cells and also in vivo using the Syrian and Roborovski hamster models. It was proven that a single-dose intranasal immunization with attenuated live viruses can elicit strong immune responses and offer complete protection against SARS-CoV-2 challenge in a robust small animal model of COVID-19.

### Vaccine design

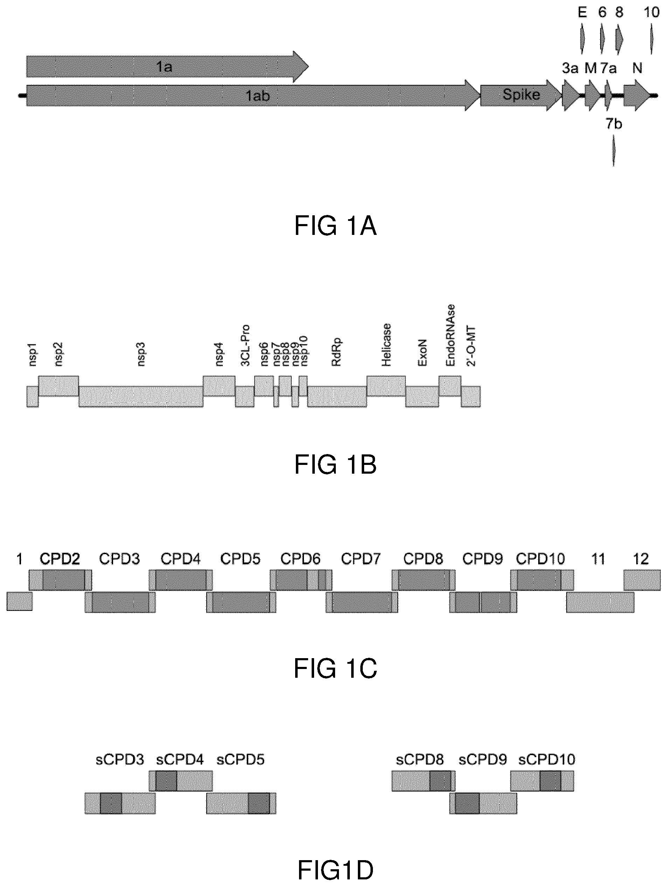

The inventors' goal was to generate attenuated SARS-CoV-2 vaccine candidates through large-scale recoding of the SARS-CoV-2 genome by CPD (Eschke et al., 2018; Groenke et al., 2020; Khedkar et al., 2018; Kunec and Osterrieder, 2016). To achieve virus attenuation in humans, the inventors recoded the genome of SARS-CoV-2 with codon pairs that are the most underrepresented in human genes (Groenke et al., 2020). The genetically modified SARS-CoV-2 mutants were produced using a recently established reverse genetics system of SARS-CoV-2 (Thi Nhu Thao et al., 2020) (Figures 1A to 1D). The system relies on 12 subgenomic fragments of the SARS-CoV-2 genome, which are assembled into a single yeast/bacterial artificial chromosome (YAC/BAC) by transformation-associated recombination (TAR) cloning in Saccharomyces cerevisiae (Noskov et al., 2002). The subgenomic fragments are approximately 3000 bp long, and the neighboring fragments overlap each other by approximately 300 bp to enable the assembly of the SARS-CoV-2 infectious clone by homologous recombination. The applied vaccine design and development is described in more detail in Trimpert et al., 2021a and Trimpert et al., 2021b.

In this context, Figures 1A to 1D illustrate the structure and recoding of the SARS-CoV-2 genome.

Figure 1A illustrates that the SARS-CoV-2 genome is a single-stranded, positive-sense RNA molecule of about 30,000 nucleotides (nt), which encodes 11 canonical ORFs. "3CL-Pro" denotes 3C-like proteinase; "RdRp" denotes RNA-dependent RNA polymerase; "ExoN" denotes 3'-to-5' exoribonuclease; "EndoRNAse" denotes endoribonuclease; and "2'-O-MT" denotes 2'-O-ribose methyltransferase.

As illustrated in Figure 1B, after infection, ORF 1a/1ab is directly translated and cleaved into 15 proteins of the replication-transcription complex.

As illustrated in Figure 1C, recoded SARS-CoV-2 mutants were constructed using a recently established reverse genetics system of SARS-CoV-2, which consists of 12 subgenomic fragments. Fragments 1, 11 and 12 were not recoded. The dark grey boxes indicate recoded sequences CPD2-10, and the light grey boxes indicate parental, non-recoded sequences in the respective fragments 2-10. The frame-shifting element contained in fragment 6 and the transcription regulatory sequence (TRS) of the spike gene in fragment 9 were excluded from the recoding process (light grey boxes present between two dark grey boxes in CPD6 and CPD9).

As shown in Figure 1D, the dark grey boxes indicate recoded sequences sCPD3-5 and sCPD8-10 in different subgenomic fragments.

To preserve the full compatibility with the available reverse genetics system, the inventors recoded only SARS-CoV-2 sequences that were not present in the overlapping parts of the subgenomic fragments (approximately 2,500 bp in each recoded fragment) (Figures 1A to 1D). This design enabled the inventors to generate a wide variety of SARS-CoV-2 mutants, carrying a single or more recoded fragments.

The inventors recoded nine fragments of the SARS-CoV-2 reverse genetics system (fragments 2-10). Fragments 1 and 12, which are relatively short, 591 and 1,812 bp respectively, and fragment 11, which contains many short ORFs, were excluded from the recoding. To ensure that mutant viruses are replication-competent, two genomic regions containing essential cis-acting RNA elements were excluded from the recoding: the frame-shifting element carried by fragment 6 and the transcription regulatory sequence (TRS) of the spike gene within fragment 9. In addition, the first 500 bp of ORF 1a located in fragment 2 were not recoded either.

### Material and Methods

### Study design

This exemplary embodiment aimed to determine the impact of removing the furin cleavage site (FCS) of sCPD9 on vaccine virus spread. The transmissibility of sCPD9-ΔFCS (i.e., a SARS-CoV-2 with a sCPD9 sequence and a deletion of the furin cleavage site) was investigated in comparison to WT (B.1) SARS-CoV-2 and sCPD9 SARS-CoV-2.

36 Syrian hamsters were kept in groups of 2 animals and housed in individually ventilated cages. Half of the respective animals received either 1*10⁵ FFU WT (B.1), sCPD9 or sCPD9-ΔFCS by intranasal instillation. Immediately after WT infection or sCPD9/sCPD9-ΔFCS vaccination, the hamsters were kept in individual cages and reunited with their non-infected/non-vaccinated conspecifics one day post infection/vaccination. Body weights were recorded daily for all hamsters and clinical conditions were checked two times per day. From 1 - 6 dpc (days post contact) mouth swabs were taken daily from contact hamsters. Contact animals were euthanized 6 dpc to assess viral loads in upper and lower respiratory tract, signs of pneumonia and seroconversion.

WT-infected and sCPD9/sCPD9-ΔFCS-vaccinated animals were challenged-infected with 1*10⁵ PFU SARS-CoV-2 Delta variant 21 days following infection/vaccination. Moreover, a group of 6 non-infected/non-vaccinated hamster was challenged with the Delta variant for comparative purposes. Subsequently, the hamsters were euthanized 2 and 5 dpch (days post challenge) to collect blood, trachea and lungs for virological, serological and histopathological analysis.

### Cells

Minimal essential medium (MEM) supplemented with 10% fetal bovine serum (FBS), 100 IU/ml penicillin G, and 100 µg/ml streptomycin was used to cultivate Vero E6 (ATCC CRL-1586) and WHO Vero RCB 10-87 cells. For Vero E6-TMPRSS2 cells (NIBSC 100978) the medium contained additionally 1000 µg/ml geneticin (G418) to select for cells expressing TMPRSS2. The cells were kept at 37 °C and 5% CO₂.

### Viruses

All SARS-CoV-2 variants used in this experiment (B.1 - BetaCoV/Munich/ChVir984/2020, B.1, EPI_ISL_406862, and Delta - B.1.617.2 SARS-CoV-2, Human, 2021, Germany ex India, 20A/452R (B.1.617) and the live attenuated SARS-CoV-2 mutants sCPD9 and sCPD9-ΔFCS were propagated on VeroE6-TMPRSS2 cells. Viruses were stored at -80 °C before experimental infection.

### Ethic statement

*In vivo* and *in vitro* experiments were carried out in the biosafety level three (BSL-3) laboratory at the Institut für Virologie, Freie Universität Berlin, Germany. Animal works were conducted according to institutional, national, and international guidelines for care and humane use of animal and authorized by the Landesamt für Gesundheit und Soziales, Berlin (permit number 0086/20).

### Animal Husbandry

Syrian hamsters (*Mesocricetus auratus;* breed RjHan:AURA), obtained from Janvier Labs at 10 weeks of age were housed in groups of 2 in individually ventilated cages. Food and water was provided *ad libitum* and cages were enriched with nesting material. The room temperature ranged constantly between 22 and 24 °C with a relative humidity of 40 to 55%. Prior to infection, the animals had 7 days to habituate to the housing conditions.

### Infection/Vaccination

Hamsters were infected or vaccinated under general anesthesia (0.15 mg/kg medetomidine, 2.0 mg/kg midazolam and butorphanol 2.5 mg/kg). 1*10⁵ FFU B.1 (Wildtype), sCPD9 or sCPD9-ΔFCS were diluted in 60 µl MEM and applied intranasally. Mock vaccinated individuals received 60 µl plain MEM without virus. 21 days after vaccination or initial infection, challenge-infection with 1*10⁵ FFU SARS-CoV-2 Delta variant was conducted as described for initial infection.

### RNA extraction and reverse transcription quantitative PCR (RT-qPCR)

Genomic copies were quantified in oropharyngeal swabs and 2.5 mg lung tissue homogenized in a bead mill (Analytic Jena). For RNA extraction the innuPREP Virus DNA/RNA Kit (Analytic Jena, Jena, Germany) was used in accordance with the manufacturer's instructions. To conduct the RT-qPCR, NEB Luna Universal Probe One-Step RT-qPCR Kit (New England Biolabs) was utilized. Cycling conditions of 10 min at 55 °C for reverse transcription, 3 min at 94 °C for activation of the enzyme and 40 cycles of 15 s at 94 °C and 30 s at 58 °C were applied on a qTower G3 cycler (Analytic Jena) using Primers and Probes as reported by Corman et al. (Corman et al., 2020).

### Plaque assay

To quantify replication-competent virus, 10-fold serial dilutions of 50 mg homogenized lung tissue were prepared and plated on Vero E6 cells grown in 12-well plates. After incubating the cells for 2.5 hours at 37 °C and 5% CO₂, cells were overlaid with 1.5 % carboxymethylcellulose sodium (Sigma Aldrich) diluted in complete growth medium. To fix the cells 72 hours after infection, PBS-buffered 4 % formaldehyde solution (pH 6.5) was used. Plaque-forming units were counted per well after staining with 0.75 % methylene blue (aqueous solution).

### Neutralization test

Serum samples from all hamsters were tested for neutralizing capacity against SARS-CoV-2 (B.1). Additionally, sera from challenge-infected animals (0, 2, 5 dpch) were tested for neutralizing activity against SARS-CoV-2 Delta variant (B.1.617). To this end, sera were inactivated for 30 minutes at 56 °C. Subsequently, two-fold serial dilutions (1:8 to 1:1024) were prepared in 96-well plates and 200 PFU of SARS-CoV-2 diluted in MEM (1 % FBS, 1 % P/S) were added to all wells. After an incubation time of 1 hour at 37 °C, the dilutions were plated on subconfluent Vero E6 cells in 96-well cell culture plates and incubated for another 72 hours. At last, plates were fixed with 4 % formaldehyde solution and stained with 0.75 % methylene blue (aqueous solution). Wells without virus-induced cytopathic effect were considered neutralized and reported as titers for the respective serum. Positive and negative controls were included in all plates.

### Histopathology

After sacrificing the animals, the left lung lobe was removed carefully and fixed for 48 hours in 4 % formaldehyde solution. Subsequently, the tissue was embedded in paraffin and cut at 2 µm thickness to stain it with hematoxylin and eosin (H&E). Lung preparation and pneumonia scoring was performed in a standardized procedure as described by Osterrieder et al. 2020.

### Growth kinetics

T25 flasks of confluent WHO Vero RCB 10-87 cells were infected with either B.1 - BetaCoV/Munich/ChVir984/2020, B.1, EPI_ISL_406862, B.1-ΔFCS, sCPD9 or sCPD9-ΔFCS at a MOI of 0.01. The virus was diluted in a final volume of 5 ml complete cell culture medium and added to each flask. After the infection, supernatant was collected at 24, 48, 72 and 96 hours post infection and subjected to one freeze-thaw cycle. Ten-fold dilutions were prepared and plated on confluent Vero E6 cells seeded in 12-well plates. Cells were overlaid with MEM containing 1.5 % carboxymethylcellulose after 1.25 hours and fixed with 4 % formaldehyde solution after 48 hours. For plaque visualization, immunofluorescence staining was conducted as described (Trimpert et al., 2021b).

### Results

### Deletion of FCS moderately increases viral titer on TMPRSS-2-negative cells

To increase the genetic stability and reduce transmissibility of sCPD9, the inventors generated an sCPD9 derivative designated sCPD9-ΔFCS that lacked the FCS in the spike protein. The sCPD9-ΔFCS mutant was engineered to contain the same deletion in the spike protein, termed 'Bristol deletion', that occurred naturally during serial passage on cultured Vero E6 cells (Davidson et al., 2020). The introduced deletion is 24 nucleotides long. It removes 9 amino acids 'NSPRRARSV' from the spike protein, including the entire FCS, and introduces isoleucine as novel amino acid at the same position instead (Davidson et al., 2020). Thus, a total of 8 amino acids is removed from the spike protein.

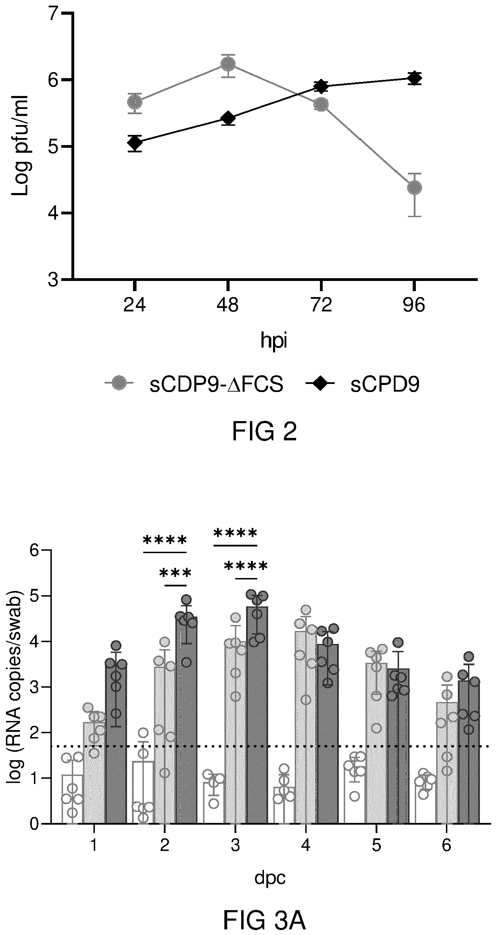

It has been widely reported that SARS-CoV-2 virus variants lacking FCS have a distinct growth advantage over viruses with intact FCS on different Vero cell lines. In accordance with these results, it was found that removal of the FCS slightly increased titers of sCPD9-ΔFCS viruses after propagation on Vero cells. Since WHO RCB 10-87 Vero cells are widely used for vaccine production, the ability to generate slightly higher peak virus titers faster on these particular cells is of great practical importance as it reduces the cost of vaccine production (Figure 2). In this context, Figure 2 shows growth kinetics of sCPD9-ΔFCS and sCPD9 conducted on WHO Vero RCB 10-87 cells in mean ± standard deviation (SD). The time points on which the supernatant was harvested are shown in hours post infection (hpi).

### Deletion of FCS prevents transmission of sCPD9-ΔFCS to unvaccinated contacts

To investigate the ability of sCPD9 and sCPD9-ΔFCS to transmit to unvaccinated contact animals, Syrian hamsters were infected with sCPD9, sCPD9-ΔFCS or parental SARS-CoV-2 (WT) on day 0. Twenty-four hours after infection, each infected hamster was placed in an individually ventilated cage with an unvaccinated hamster. Already on the first day of this cohabitation, swabs from the mouths of all naive animals that were in contact with WT-infected animals were strongly positive for SARS-CoV-2 RNA. All naive animals that were in contact with sCPD9-vaccinated subjects contracted the virus on days 1-3 of cohabitation and displayed a similar, although delayed, course of virus replication in the upper airways when compared with WT-infected animals. In contrast, naive animals in contact with sCPD9-ΔFCS vaccinated subjects did not become positive for SARS-CoV-2 RNA during the first 7 days of cohabitation which was the duration of the observation period (Figure 3A). In this context, Figure 3A shows genomic RNA (gRNA) copies found in mouth swabs taken daily (1-6 dpc) from contact hamsters in mean ± SD. Two-way ANOVA and Tukey's multiple comparisons test were performed (* p < 0.05, ** p < 0.01, *** p < 0.001, and **** p < 0.0001). In this and in all Figures, non-filled circles or squares (first bar from the left) denote sCPD9-ΔFCS, light grey circles or squares (second bar from the left) denote sCPD9, and dark grey circles or squares (third bar from the left) denote B.1 (WT SARS-CoV-2).

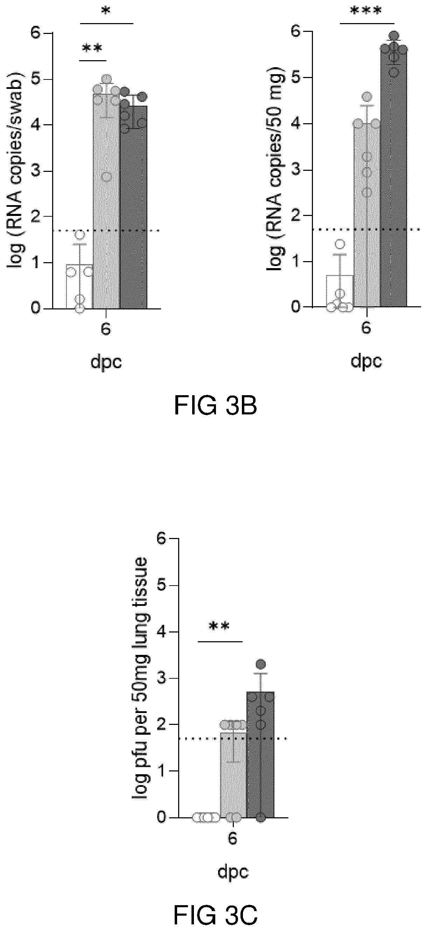

Natural transmission of WT SARS-CoV-2 caused the expected COVID-19-like pneumonia in contact animals as evidenced by histological examination on day 7 of cohabitation (Figure 3B). In agreement with previous findings, the inflammatory changes in the lungs were greatly attenuated in animals that contracted sCPD9. In addition, the lungs of animals cohabitating with the sCPD9-ΔFCS vaccinated animals showed virtually no signs of inflammation.

### sCPD9-ΔFCS is highly attenuated in Syrian hamsters

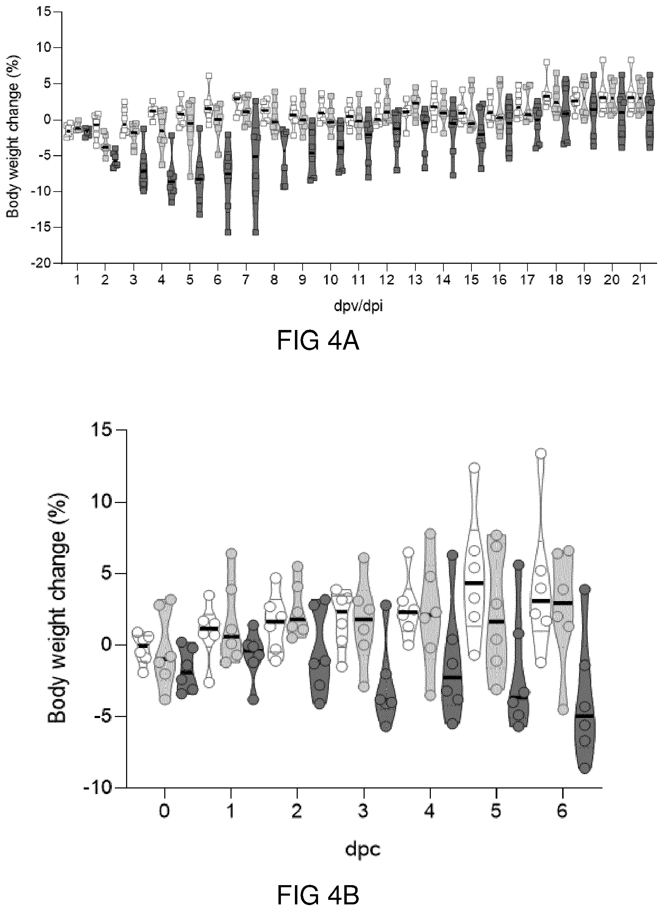

All hamsters remained clinically healthy after infection with sCPD9 or sCPD9-ΔFCS, whereas WT-infected animals showed the expected mild to moderate signs of disease, such as forced breathing and considerable weight loss during the first week after infection (Figure 4A). In this context, Figure 4A shows body weight loss in percentage after initial vaccination/infection shown per group and up to challenge-infection time point 21 dpi/dpv (dpi = days post infection, dpv = days post vaccination).

However, in the absence of other visible signs of disease, sCPD9-infected animals showed a trend towards slightly decreasing body weights while sCPD9-ΔFCS-vaccinated animals presented with relatively stable weights in the week following vaccination.

Although all contact animals that were exposed to sCPD9- or WT-infected animals contracted the respective virus, clinical signs of disease and body weight loss occurred only in animals that contracted the WT virus (Figure 4B, illustrating weight development of contact animals in percent per group during the co-housing period). Both Figure 4A and Figure 4B show violin plots (truncated) with quartiles and median.

Similarly, severe histological signs of lung inflammation on day 7 post contact, were present only in contact animals that contracted the WT virus (Figure 3B, illustrating the number of gRNA copies recovered from oropharyngeal swabs and homogenized lungs and replication-competent virus detected in lung tissue; the limit of detection is marked by dotted lines). The data depicted in Figures 3B to 3D was statistically evaluated by conducting Kruskal-Wallis test and Dunn's multiple comparisons test (* p < 0.05, ** p < 0.01, *** p < 0.001, and **** p < 0.0001 ).

Figure 3C shows the replication-competent virus detected in lung tissue. The limit of detection is again marked by dotted lines.

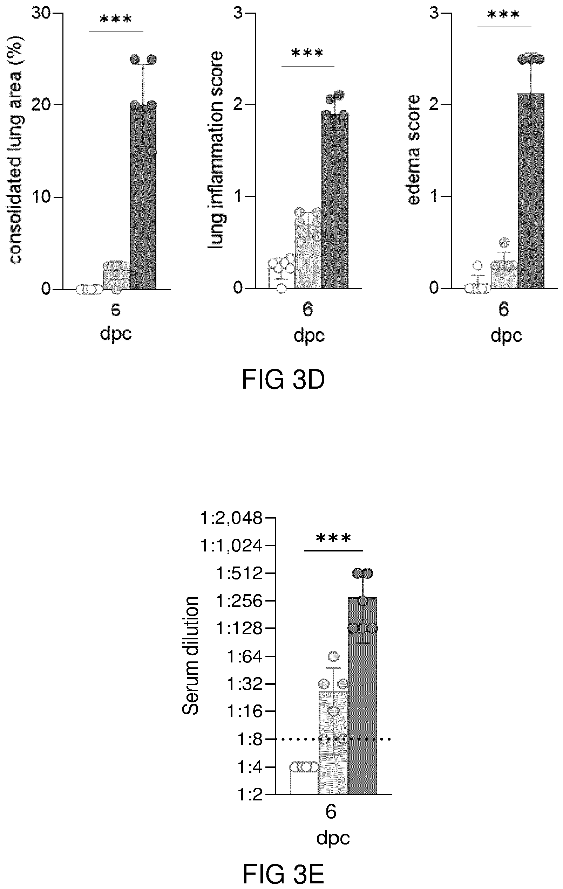

Figure 3D shows the histopathological scoring including consolidated lung area in percentage, lung inflammation score with severity of pneumonia, influx of neutrophils, lymphocytes and macrophages, bronchial epithelial necrosis, bronchitis, alveolar epithelial necrosis, perivascular lymphocyte cuffs and pneumocyte type II hyperplasia and edema score accounting for perivascular edema and alveolar edema.

Figure 3E shows the neutralizing activity against SARS-CoV-2 B.1 WT of sera taken from contact hamsters at day 6 after contact.

Contact animals that became infected with sCPD9 showed only mild inflammatory changes in the lungs, these further reduced or absent in the lungs of the sCPD9-ΔFCS-infected contact animals which had not contracted the virus.

### Deletion of FCS does not reduce vaccine efficacy

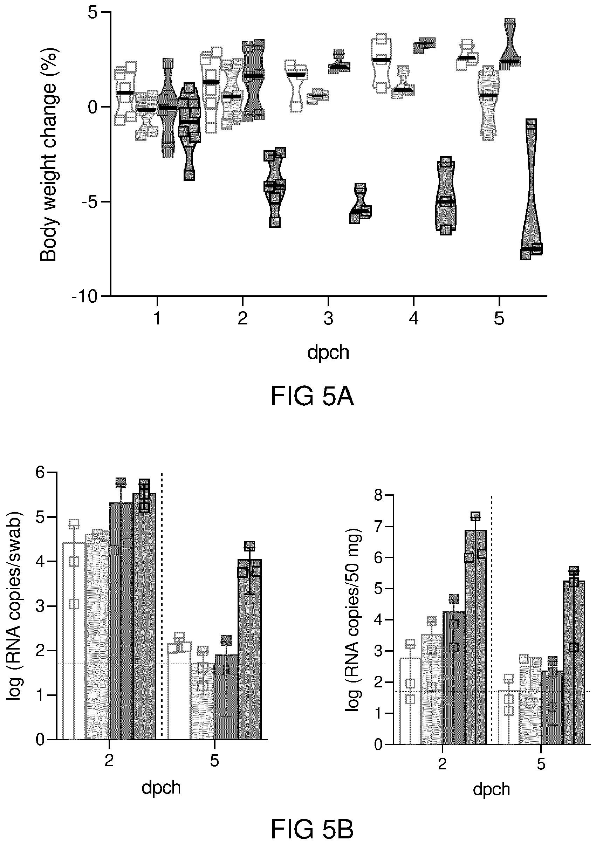

All infected animals were challenged with the pathogenic SARS-CoV-2 Delta variant on day 21 after infection. None of the challenged animals developed clinical signs of disease or exhibited marked body weight loss which was observed in the unvaccinated control animals (Figure 5A, illustrating body weight changes that were controlled until analysis time points 2 + 5 dpch and are shown in percent per group). In this and all other Figures, medium grey squares (fourth bar from the left) denote mock-vaccinated animals.

As expected, protection against replication of the challenge virus was comparable in all three infected groups. On day 2 after challenge, all groups showed high viral RNA loads in the upper respiratory tract (Figure 5B, illustrating quantification of gRNA in oropharyngeal swabs and lung and infectious virus particles detected in homogenized lung tissue). In Figures 5B to 5E, dotted lines mark the limit of detection. In addition, the data presented in Figures 5B to 5E was statistically analyzed by two-way ANOVA and Tukey's multiple comparisons test (* p < 0.05, ** p < 0.01, *** p < 0.001, and **** p < 0.0001). The observed high viral RNA loads, however, were reduced to levels near the detection limit by day 5 after challenge in all studied groups. Protection in the lower respiratory tract was more pronounced at day 2 after challenge with significantly lower viral RNA loads and minimal levels of replication-competent virus in the lung of infected animals (Figure 5B). By day 5 after challenge, RNA loads were near or below the detection limit, and no replicating virus was present in the lungs of any of the challenged subjects.

Overall, virological parameters confirmed high protective efficacy of sCPD9 and sCPD9-ΔFCS vaccine candidates and were comparable to those of the WT virus.

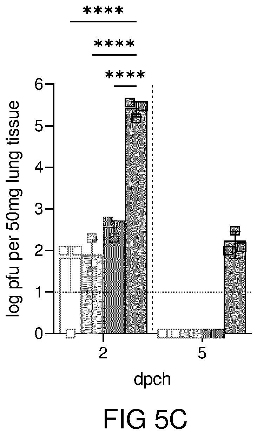

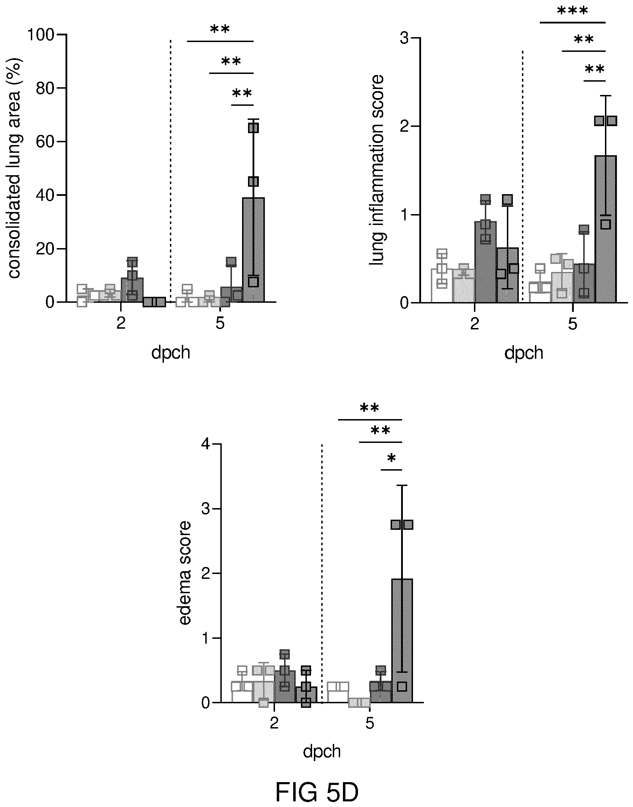

Protective efficacy of sCPD9 and sCPD9-ΔFCS vaccine viruses was determined by examining the lung histopathology of uninfected and sCPD9-, sCPD9-ΔFCS- and WT-infected hamsters on days 2 and 5 after challenge infection (Figure 5C, illustrating infectious virus particles detected in homogenized lung tissue, and Figure 5D, illustrating the percentage of lung area consolidated by SARS-CoV-2 infection; lung inflammation score accounts for severity of pneumonia, influx of neutrophils, lymphocytes and macrophages, bronchial epithelial necrosis, bronchitis, alveolar epithelial necrosis, perivascular lymphocyte cuffs and pneumocyte type II hyperplasia; pulmonary edema is scored based on the extent of perivascular and alveolar edema). Here, it became evident that WT-infected animals were more prone to develop inflammation and showed a tendency towards increased influx of immune cells compared with animals that were infected with sCPD9 or sCPD9-ΔFCS viruses or unvaccinated animals. This situation was ameliorated by day 5 after challenge, suggesting that immune system of the WT-infected animals transiently overreacted shortly after challenge, causing pulmonary damage that was observed on day 2 after challenge. Importantly, all animals showed excellent and comparable protection against COVID-19-like pneumonia at all examined time points after challenge.

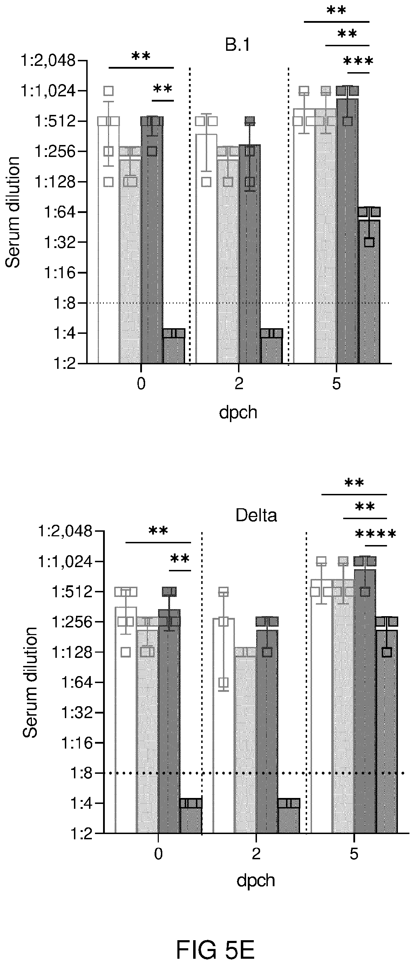

Finally, the humoral immune responses were compared against SARS-CoV-2 WT and the Delta variant in infected animals before challenge and on days 2 or 5 after challenge (Figure 5E, illustrating neutralizing activity against B.1 and Delta variant of hamster sera taken on day 0 (prior challenge), 2 and 5 after SARS-CoV-2 Delta challenge; the range of detection reached from dilution 1:8 to 1 :1024). The results showed that vaccination/infection with sCPD9, sCPD9-ΔFCS or WT viruses induced comparable levels of neutralizing antibodies at all time points tested.

### sCPD9-ΔFCS is slightly more attenuated than sCPD9, but equally protective

The results presented here indicate that removal of the FCS from sCPD9 results in a non-transmissible and highly attenuated virus in the Syrian hamster model. Despite the high level of attenuation, the sCPD9-ΔFCS virus showed equal protection against SARS-CoV-2 variant Delta as the parental sCPD9 virus that has an intact FCS in the spike protein. In terms of virological, serological and histological parameters, both sCPD9 and sCPD9-ΔFCS vaccine viruses provided excellent and comparable protection against virus replication and disease. Overall, protection conferred by either vaccine virus was comparable to that of the induced by infection with WT virus. Remarkably, WT-infected animals showed stronger signs of inflammation in the lungs during first days after challenge.

Thorough histological examination revealed another remarkable advantage of a vaccination with sCPD9-ΔFCS or sCPD9 over vaccination with WT virus with respect to subsequent virus challenge infection. A previous WT virus infection and subsequent challenge with SARS-CoV-2 led to a significant alveolar bronchiolysis in tested animals. In contrast, no alveolar bronchiolysis was observed in animals that were challenged with SARS-CoV-2 after prior vaccination with sCPD9-ΔFCS or sCPD9.

### Benefits of FCS removal for mass production of LAV SARS-CoV-2 vaccines

Many reports have shown that FCS impairs replication of SARS-CoV-2 in TMPRSS2-deficient cells such as Vero or Vero-E6 cells, because SARS-CoV-2 variants lacking a functional FCS become rapidly dominant upon passage of TMPRSS2-deficient cells (Davidson et al., 2020; Klimstra et al., 2020; Lau et al., 2020; Liu et al., 2020; Ogando et al., 2020; Sasaki et al., 2021b; Wong et al., 2021). These results suggest that mutant viruses lacking the functional FCS have a strong selection advantage for replication in cells that do not express TMPRSS2. The results presented here have confirmed these observations, as the sCPD9-ΔFCS mutant replicated to higher titers than the original sCPD9 virus in Vero cells.

Thus, removal of the FCS from SARS-CoV-2-LAV has two advantages for LAV vaccine production. Because most vaccine manufacturers use Vero cells to produce LAV vaccines, increasing viral titers also helps reduce the production costs, but more importantly, it facilitates purification of sufficient numbers of infectious virus particles that are required for a single vaccination.

The second major advantage of removing FCS is that it greatly improves the genetic stability of the LAV vaccine candidate. One of the prerequisites for the use of LAV vaccines in humans is that the vaccine virus population has a high degree of genetic homogeneity. Since SARS-CoV-2 rapidly loses its FCS at the S1/S2 boundary after passage into Vero cells, removal of the FCS eliminates the problem of genetic instability, and the vaccine virus can be produced with high genetic homogeneity.

### LIST OF REFERENCES CITED IN THE PRECEDING SECTIONS

1. Johnson & Johnson COVID-19 Vaccine Authorized by U.S. FDA For Emergency Use - First Single-Shot Vaccine in Fight Against Global Pandemic https://www.jnj.com/johnson-johnson-covid-19-vaccine-authorized-by-u-s-fda-for-emergency-usefirst-single-shot-vaccine-in-fight-against-global-pandemic#_edn2 (Johnson & Johnson).
2. The World Health Organization. Draft landscape of COVID-19 candidate vaccines. https://www.who.int/publications/m/item/draft-landscape-of-covid-19-candidate-vaccines (WHO).
3. Abu-Raddad, L.J., Chemaitelly, H., Butt, A.A., and National Study Group for, C.-V. (2021). Effectiveness of the BNT162b2 Covid-19 Vaccine against the B.1.1.7 and B.1.351 Variants. N Engl J Med.
4. Almazan, F., Gonzalez, J.M., Penzes, Z., Izeta, A., Calvo, E., Plana-Duran, J., and Enjuanes, L. (2000). Engineering the largest RNA virus genome as an infectious bacterial artificial chromosome. Proceedings of the National Academy of Sciences of the United States of America 97, 5516-5521.
5. Anderson, E.J., Rouphael, N.G., Widge, A.T., Jackson, L.A., Roberts, P.C., Makhene, M., Chappell, J.D., Denison, M.R., Stevens, L.J., Pruijssers, A.J., et al. (2020). Safety and Immunogenicity of SARS-CoV-2 mRNA-1273 Vaccine in Older Adults. N Engl J Med 383, 2427-2438.
6. Baden, L.R., EI Sahly, H.M., Essink, B., Kotloff, K., Frey, S., Novak, R., Diemert, D., Spector, S.A., Rouphael, N., Creech, C.B., et al. (2021). Efficacy and Safety of the mRNA-1273 SARS-CoV-2 Vaccine. N Engl J Med 384, 403-416.
7. Bazin, H. (2003). A brief history of the prevention of infectious diseases by immunisations. Comparative immunology, microbiology and infectious diseases 26, 293-308.
8. Broadbent, A.J., Santos, C.P., Anafu, A., Wimmer, E., Mueller, S., and Subbarao, K. (2016). Evaluation of the attenuation, immunogenicity, and efficacy of a live virus vaccine generated by codon-pair bias de-optimization of the 2009 pandemic H1N1 influenza virus, in ferrets. Vaccine 34, 563-570.
9. S. Cele, I. Gazy, L. Jackson, S. H. Hwa, H. Tegally, G. Lustig, J. Giandhari, S. Pillay, E. Wilkinson, Y. Naidoo, et al., A. Network for Genomic Surveillance in South, C.-K. Team, R. J. Lessells, T. de Oliveira, A. Sigal, Escape of SARS-CoV-2 501Y.V2 from neutralization by convalescent plasma. Nature 593, 142-146 (2021).
10. Chan, J.F., Yuan, S., Kok, K.H., To, K.K., Chu, H., Yang, J., Xing, F., Liu, J., Yip, C.C., Poon, R.W., et al. (2020). A familial cluster of pneumonia associated with the 2019 novel coronavirus indicating person-to-person transmission: a study of a family cluster. Lancet 395, 514-523.
11. Chen, N., Zhou, M., Dong, X., Qu, J., Gong, F., Han, Y., Qiu, Y., Wang, J., Liu, Y., Wei, Y., et al. (2020). Epidemiological and clinical characteristics of 99 cases of 2019 novel coronavirus pneumonia in Wuhan, China: a descriptive study. Lancet 395, 507-513.
12. Coleman, J.R., Papamichail, D., Skiena, S., Futcher, B., Wimmer, E., and Mueller, S. (2008). Virus attenuation by genome-scale changes in codon pair bias. Science 320, 1784-1787.
13. Corbett, K.S., Flynn, B., Foulds, K.E., Francica, J.R., Boyoglu-Barnum, S., Werner, A.P., Flach, B., O'Connell, S., Bock, K.W., Minai, M., et al. (2020). Evaluation of the mRNA-1273 Vaccine against SARS-CoV-2 in Nonhuman Primates. N Engl J Med 383, 1544-1555.
14. Corman, V.M., Landt, O., Kaiser, M., Molenkamp, R., Meijer, A., Chu, D.K., Bleicker, T., Brunink, S., Schneider, J., Schmidt, M.L., et al. (2020). Detection of 2019 novel coronavirus (2019-nCoV) by real-time RT-PCR. Euro Surveill 25.
15. Dagan, N., Barda, N., Kepten, E., Miron, O., Perchik, S., Katz, M.A., Hernan, M.A., Lipsitch, M., Reis, B., and Balicer, R.D. (2021). BNT162b2 mRNA Covid-19 Vaccine in a Nationwide Mass Vaccination Setting. N Engl J Med 384, 1412-1423.
16. N. G. Davies, S. Abbott, R. C. Barnard, C. I. Jarvis, A. J. Kucharski, J. D. Munday, C. A. B. Pearson, T. W. Russell, D. C. Tully, A. D. Washburne, T. Wenseleers, A. Gimma, W. Waites, K. L. M. Wong, K. van Zandvoort, J. D. Silverman, C. C.-W. Group, C.-G. U. Consortium, K. Diaz-Ordaz, R. Keogh, R. M. Eggo, S. Funk, M. Jit, K. E. Atkins, W. J. Edmunds, Estimated transmissibility and impact of SARS-CoV-2 lineage B.1.1.7 in England. Science 372, (2021).
17. W. Dejnirattisai, D. Zhou, P. Supasa, C. Liu, A. J. Mentzer, H. M. Ginn, Y. Zhao, H. M. E. Duyvesteyn, A. Tuekprakhon, R. Nutalai, et al., Antibody evasion by the P.1 strain of SARS-CoV-2. Cell 184, 2939-2954 e2939 (2021).
18. Dong, E., Du, H., and Gardner, L. (2020). An interactive web-based dashboard to track COVID-19 in real time. Lancet Infect Dis 20, 533-534.
19. O. Dyer, Covid-19: Delta infections threaten herd immunity vaccine strategy. BMJ 374, n1933 (2021 ).
20. Ella, R., Reddy, S., Jogdand, H., Sarangi, V., Ganneru, B., Prasad, S., Das, D., Raju, D., Praturi, U., Sapkal, G., et al. (2021). Safety and immunogenicity of an inactivated SARS-CoV-2 vaccine, BBV152: interim results from a double-blind, randomised, multicentre, phase 2 trial, and 3-month follow-up of a double-blind, randomised phase 1 trial. Lancet Infect Dis.
21. Emary, K.R.W., Golubchik, T., Aley, P.K., Ariani, C.V., Angus, B., Bibi, S., Blane, B., Bonsall, D., Cicconi, P., Charlton, S., et al. (2021). Efficacy of ChAdOx1 nCoV-19 (AZD1222) vaccine against SARS-CoV-2 variant of concern 202012/01 (B.1.1.7): an exploratory analysis of a randomised controlled trial. Lancet 397, 1351-1362.
22. Eschke, K., Trimpert, J., Osterrieder, N., and Kunec, D. (2018). Attenuation of a very virulent Marek's disease herpesvirus (MDV) by codon pair bias deoptimization. PLoS Pathog 14, e1006857.
23. N. R. Faria, T. A. Mellan, C. Whittaker, I. M. Claro, D. D. S. Candido, S. Mishra, M. A. E. Crispim, F. C. S. Sales, I. Hawryluk, J. T. McCrone et al., Genomics and epidemiology of the P.1 SARS-CoV-2 lineage in Manaus, Brazil. Science 372, 815-821 (2021).
24. Gao, Q., Bao, L., Mao, H., Wang, L., Xu, K., Yang, M., Li, Y., Zhu, L., Wang, N., Lv, Z., et al. (2020). Development of an inactivated vaccine candidate for SARS-CoV-2. Science 369, 77-81.
25. Garg, S., Kim, L., Whitaker, M., O'Halloran, A., Cummings, C., Holstein, R., Prill, M., Chai, S.J., Kirley, P.D., Alden, N.B., et al. (2020). Hospitalization Rates and Characteristics of Patients Hospitalized with Laboratory-Confirmed Coronavirus Disease 2019 - COVID-NET, 14 States, March 1-30, 2020. MMWR Morb Mortal Wkly Rep 69, 458-464.
26. Greinacher, A., Thiele, T., Warkentin, T.E., Weisser, K., Kyrle, P.A., and Eichinger, S. (2021). Thrombotic Thrombocytopenia after ChAdOx1 nCov-19 Vaccination. N Engl J Med.
27. Groenke, N., Trimpert, J., Merz, S., Conradie, A.M., Wyler, E., Zhang, H., Hazapis, O.G., Rausch, S., Landthaler, M., Osterrieder, N., et al. (2020). Mechanism of Virus Attenuation by Codon Pair Deoptimization. Cell Rep 31, 107586.
28. Gruber, A.D., Osterrieder, N., Bertzbach, L.D., Vladimirova, D., Greuel, S., Ihlow, J., Horst, D., Trimpert, J., and Dietert, K. (2020). Standardization of Reporting Criteria for Lung Pathology in SARS-CoV-2 Infected Hamsters - What Matters? Am J Respir Cell Mol Biol 63, 856-859.
29. G. A. Gutman, G. W. Hatfield, Nonrandom utilization of codon pairs in Escherichia coli. Proc Natl Acad Sci U S A 86, 3699-3703 (1989).
30. Haas, E.J., Angulo, F.J., McLaughlin, J.M., Anis, E., Singer, S.R., Khan, F., Brooks, N., Smaja, M., Mircus, G., Pan, K., et al. (2021). Impact and effectiveness of mRNA BNT162b2 vaccine against SARS-CoV-2 infections and COVID-19 cases, hospitalisations, and deaths following a nationwide vaccination campaign in Israel: an observational study using national surveillance data. Lancet.
31. Hall, V.J., Foulkes, S., Saei, A., Andrews, N., Oguti, B., Charlett, A., Wellington, E., Stowe, J., Gillson, N., Atti, A., et al. (2021). COVID-19 vaccine coverage in health-care workers in England and effectiveness of BNT162b2 mRNA vaccine against infection (SIREN): a prospective, multicentre, cohort study. Lancet.
32. M. D. T. Hitchings, O. T. Ranzani, M. S. Scaramuzzini Torres, S. B. de Oliveira, M. Almiron, R. Said, R. Borg, W. L. Schulz, R. D. de Oliveira, P. V. da Silva, et al., Effectiveness of CoronaVac in the setting of high SARS-CoV-2 P.1 variant transmission in Brazil: A test-negative case-control study. medRxiv, 2021.2004.2007.21255081 (2021).
33. Y. H. Jang, B. L. Seong, Immune Responses Elicited by Live Attenuated Influenza Vaccines as Correlates of Universal Protection against Influenza Viruses. Vaccines (Basel) 9, (2021).
34. Jackson, L.A., Anderson, E.J., Rouphael, N.G., Roberts, P.C., Makhene, M., Coler, R.N., McCullough, M.P., Chappell, J.D., Denison, M.R., Stevens, L.J., et al. (2020). An mRNA Vaccine against SARS-CoV-2 - Preliminary Report. N Engl J Med.
35. Kew, O.M., Sutter, R.W., de Gourville, E.M., Dowdle, W.R., and Pallansch, M.A. (2005). Vaccine-derived polioviruses and the endgame strategy for global polio eradication. Annu Rev Microbiol 59, 587-635.
36. Khedkar, P.H., Osterrieder, N., and Kunec, D. (2018). Codon pair bias deoptimization of the major oncogene meq of a very virulent Marek's disease virus. J Gen Virol 99, 1705-1716.
37. Kunec, D., and Osterrieder, N. (2016). Codon Pair Bias Is a Direct Consequence of Dinucleotide Bias. Cell Rep 14, 55-67.
38. Kusters, I., and Almond, J.W. (2008). Vaccine Strategies. In Encyclopedia of Virology (Third Edition), B.W.J.M. Editors-in-Chief: , and M.H.V.v. Regenmortel, eds. (Oxford: Academic Press), pp. 235-243.
39. Kutter, J.S., de Meulder, D., Bestebroer, T.M., Lexmond, P., Mulders, A., Richard, M., Fouchier, R.A.M., and Herfst, S. (2021). SARS-CoV and SARS-CoV-2 are transmitted through the air between ferrets over more than one meter distance. Nat Commun 12, 1653.
40. Lauring, A.S., Acevedo, A., Cooper, S.B., and Andino, R. (2012). Codon usage determines the mutational robustness, evolutionary capacity, and virulence of an RNA virus. Cell Host Microbe 12, 623-632.
41. Lauring, A.S., Jones, J.O., and Andino, R. (2010). Rationalizing the development of live attenuated virus vaccines. Nature biotechnology 28, 573-579.
42. Le Nouen, C., Brock, L.G., Luongo, C., McCarty, T., Yang, L., Mehedi, M., Wimmer, E., Mueller, S., Collins, P.L., Buchholz, U.J., et al. (2014). Attenuation of human respiratory syncytial virus by genome-scale codon-pair deoptimization. Proceedings of the National Academy of Sciences of the United States of America 111, 13169-13174.
43. C. Liu, H. M. Ginn, W. Dejnirattisai, P. Supasa, B. Wang, A. Tuekprakhon, R. Nutalai, D. Zhou, A. J. Mentzer, Y. Zhao, et al., Reduced neutralization of SARS-CoV-2 B.1.617 by vaccine and convalescent serum. Cell, (2021).
44. Logunov, D.Y., Dolzhikova, I.V., Shcheblyakov, D.V., Tukhvatulin, A.I., Zubkova, O.V., Dzharullaeva, A.S., Kovyrshina, A.V., Lubenets, N.L., Grousova, D.M., Erokhova, A.S., et al. (2021). Safety and efficacy of an rAd26 and rAd5 vector-based heterologous prime-boost COVID-19 vaccine: an interim analysis of a randomised controlled phase 3 trial in Russia. Lancet 397, 671-681.
45. Madhi, S.A., Baillie, V., Cutland, C.L., Voysey, M., Koen, A.L., Fairlie, L., Padayachee, S.D., Dheda, K., Barnabas, S.L., Bhorat, Q.E., et al. (2021). Efficacy of the ChAdOx1 nCoV-19 Covid-19 Vaccine against the B.1.351 Variant. N Engl J Med.
46. Mallapaty, S. (2021). China's COVID vaccines are going global - but questions remain. Nature.
47. J. P. Moore, P. A. Offit, SARS-CoV-2 Vaccines and the Growing Threat of Viral Variants. JAMA 325, 821-822 (2021).
48. Mueller, S., Coleman, J.R., Papamichail, D., Ward, C.B., Nimnual, A., Futcher, B., Skiena, S., and Wimmer, E. (2010). Live attenuated influenza virus vaccines by computer-aided rational design. Nature biotechnology 28, 723-726.
49. Mulligan, M.J., Lyke, K.E., Kitchin, N., Absalon, J., Gurtman, A., Lockhart, S., Neuzil, K., Raabe, V., Bailey, R., Swanson, K.A., et al. (2020). Phase I/II study of COVID-19 RNA vaccine BNT162b1 in adults. Nature.
50. Nakamura, T., Karakida, N., Dantsuka, A., Ichii, O., Elewa, Y.H.A., Kon, Y., Nagasaki, K.I., Hattori, H., and Yoshiyasu, T. (2017). Effects of a mixture of medetomidine, midazolam and butorphanol on anesthesia and blood biochemistry and the antagonizing action of atipamezole in hamsters. J Vet Med Sci 79, 1230-1235.
51. F. G. Naveca, V. Nascimento, V. C. de Souza, A. L. Corado, F. Nascimento, G. Silva, A. Costa, D. Duarte, K. Pessoa, M. Mejia, M. J. Brandao, M. Jesus, L. Goncalves, C. F. da Costa, V. Sampaio, D. Barros, M. Silva, T. Mattos, G. Pontes, L. Abdalla, J. H. Santos, I. Arantes, F. Z. Dezordi, M. M. Siqueira, G. L. Wallau, P. C. Resende, E. Delatorre, T. Graf, G. Bello, COVID-19 in Amazonas, Brazil, was driven by the persistence of endemic lineages and P.1 emergence. Nat Med, (2021).
52. Noskov, V., Kouprina, N., Leem, S.H., Koriabine, M., Barrett, J.C., and Larionov, V. (2002). A genetic system for direct selection of gene-positive clones during recombinational cloning in yeast. Nucleic Acids Res 30, E8.
53. Odon, V., Fros, J.J., Goonawardane, N., Dietrich, I., Ibrahim, A., Alshaikhahmed, K., Nguyen, D., and Simmonds, P. (2019). The role of ZAP and OAS3/RNAseL pathways in the attenuation of an RNA virus with elevated frequencies of CpG and UpA dinucleotides. Nucleic Acids Res 47, 8061 - 8083.
54. Osterrieder, N., Bertzbach, L.D., Dietert, K., Abdelgawad, A., Vladimirova, D., Kunec, D., Hoffmann, D., Beer, M., Gruber, A.D., and Trimpert, J. (2020). Age-Dependent Progression of SARS-CoV-2 Infection in Syrian Hamsters. Viruses 12.
55. Osterrieder, N., and Kunec, D. (2018). Attenuation of Viruses by Large-Scale Recoding of their Genomes: the Selection Is Always Biased. Curr Clin Microbiol Rep 5, 66-72.
56. D. Planas, T. Bruel, L. Grzelak, F. Guivel-Benhassine, I. Staropoli, F. Porrot, C. Planchais, J. Buchrieser, M. M. Rajah, E. Bishop, et al., Sensitivity of infectious SARS-CoV-2 B.1.1.7 and B.1.351 variants to neutralizing antibodies. Nat Med 27, 917-924 (2021a).
57. D. Planas, D. Veyer, A. Baidaliuk, I. Staropoli, F. Guivel-Benhassine, M. M. Rajah, C. Planchais, F. Porrot, N. Robillard, J. Puech, et al., Reduced sensitivity of SARS-CoV-2 variant Delta to antibody neutralization. Nature, (2021b).
58. Polack, F.P., Thomas, S.J., Kitchin, N., Absalon, J., Gurtman, A., Lockhart, S., Perez, J.L., Perez Marc, G., Moreira, E.D., Zerbini, C., et al. (2020). Safety and Efficacy of the BNT162b2 mRNA Covid-19 Vaccine. N Engl J Med.
59. Sahin, U., Muik, A., Derhovanessian, E., Vogler, I., Kranz, L.M., Vormehr, M., Baum, A., Pascal, K., Quandt, J., Maurus, D., et al. (2020). COVID-19 vaccine BNT162b1 elicits human antibody and TH1 T-cell responses. Nature.
60. Schneider, C.A., Rasband, W.S., and Eliceiri, K.W. (2012). NIH Image to ImageJ: 25 years of image analysis. Nat Methods 9, 671-675.
61. Shen, S.H., Stauft, C.B., Gorbatsevych, O., Song, Y., Ward, C.B., Yurovsky, A., Mueller, S., Futcher, B., and Wimmer, E. (2015). Large-scale recoding of an arbovirus genome to rebalance its insect versus mammalian preference. Proceedings of the National Academy of Sciences of the United States of America.
62. V. Shinde, S. Bhikha, Z. Hoosain, M. Archary, Q. Bhorat, L. Fairlie, U. Lalloo, M. S. L. Masilela, D. Moodley, S. Hanley, et al., Efficacy of NVX-CoV2373 Covid-19 Vaccine against the B.1.351 Variant. N Engl J Med 384, 1899-1909 (2021).
63. Sia, S.F., Yan, L.-M., Chin, A.W.H., Fung, K., Poon, L.L.M., Nicholls, J.M., Peiris, M., and Yen, H.-L. (2020). Pathogenesis and transmission of SARS-CoV-2 virus in golden Syrian hamsters.
64. Solforosi, L., Kuipers, H., Jongeneelen, M., Rosendahl Huber, S.K., van der Lubbe, J.E.M., Dekking, L., Czapska-Casey, D.N., Izquierdo Gil, A., Baert, M.R.M., Drijver, J., et al. (2021). Immunogenicity and efficacy of one and two doses of Ad26.COV2.S COVID vaccine in adult and aged NHP. J Exp Med 218.
65. Song, Y., Liu, Y., Ward, C.B., Mueller, S., Futcher, B., Skiena, S., Paul, A.V., and Wimmer, E. (2012). Identification of two functionally redundant RNA elements in the coding sequence of poliovirus using computer-generated design. Proceedings of the National Academy of Sciences of the United States of America 109, 14301-14307.
66. H. Tegally, E. Wilkinson, R. J. Lessells, J. Giandhari, S. Pillay, N. Msomi, K. Mlisana, J. N. Bhiman, A. von Gottberg, S. Walaza, V. Fonseca, M. Allam, A. Ismail, A. J. Glass, S. Engelbrecht, G. Van Zyl, W. Preiser, C. Williamson, F. Petruccione, A. Sigal, I. Gazy, D. Hardie, N. Y. Hsiao, D. Martin, D. York, D. Goedhals, E. J. San, M. Giovanetti, J. Lourenco, L. C. J. Alcantara, T. de Oliveira, Sixteen novel lineages of SARS-CoV-2 in South Africa. Nat Med 27, 440-446 (2021).
67. Thi Nhu Thao, T., Labroussaa, F., Ebert, N., V'Kovski, P., Stalder, H., Portmann, J., Kelly, J., Steiner, S., Holwerda, M., Kratzel, A., et al. (2020). Rapid reconstruction of SARS-CoV-2 using a synthetic genomics platform. Nature 582, 561-565.
68. Trimpert, J., Adler, J.M., Eschke, K., Abdelgawad, A., Firsching, T.C., Ebert, N., Thao, T.T.N., Gruber, A.D., Thiel, V., Osterrieder, N., et al. (2021a). Live attenuated virus vaccine protects against SARS-CoV-2 variants of concern B.1.1.7 (Alpha) and B.1.351 (Beta). Sci Adv 7, eabk0172.
69. Trimpert, J., Dietert, K., Firsching, T.C., Ebert, N., Thi Nhu Thao, T., Vladimirova, D., Kaufer, S., Labroussaa, F., Abdelgawad, A., Conradie, A., et al. (2021b). Development of safe and highly protective live-attenuated SARS-CoV-2 vaccine candidates by genome recoding. Cell Rep 36, 109493.
70. Trimpert, T., Vladimirova, D., Dietert, K., Abdelgawad, A., Kunec, D., Dökel, D., Gruber, A.D., Bertzbach, L., and Osterrieder, N. (2020). The Roborovski dwarf hamster - a highly susceptible model for a rapid and fatal course of SARS-CoV-2 infection. Cell Reports 33, 108488.
71. E. Volz, S. Mishra, M. Chand, J. C. Barrett, R. Johnson, L. Geidelberg, W. R. Hinsley, D. J. Laydon, G. Dabrera, A. O'Toole, R. Amato, M. Ragonnet-Cronin, I. Harrison, B. Jackson, C. V. Ariani, O. Boyd, N. J. Loman, J. T. McCrone, S. Goncalves, D. Jorgensen, R. Myers, V. Hill, D. K. Jackson, K. Gaythorpe, N. Groves, J. Sillitoe, D. P. Kwiatkowski, C.-G. U. consortium, S. Flaxman, O. Ratmann, S. Bhatt, S. Hopkins, A. Gandy, A. Rambaut, N. M. Ferguson, Assessing transmissibility of SARS-CoV-2 lineage B.1.1.7 in England. Nature 593, 266-269 (2021).
72. Voysey, M., Clemens, S.A.C., Madhi, S.A., Weckx, L.Y., Folegatti, P.M., Aley, P.K., Angus, B., Baillie, V.L., Barnabas, S.L., Bhorat, Q.E., et al. (2021a). Safety and efficacy of the ChAdOx1 nCoV-19 vaccine (AZD1222) against SARS-CoV-2: an interim analysis of four randomised controlled trials in Brazil, South Africa, and the UK. Lancet 397, 99-111.
73. Voysey, M., Costa Clemens, S.A., Madhi, S.A., Weckx, L.Y., Folegatti, P.M., Aley, P.K., Angus, B., Baillie, V.L., Barnabas, S.L., Bhorat, Q.E., et al. (2021b). Single-dose administration and the influence of the timing of the booster dose on immunogenicity and efficacy of ChAdOx1 nCoV-19 (AZD1222) vaccine: a pooled analysis of four randomised trials. Lancet 397, 881-891.
74. Walsh, E.E., Frenck, R.W., Jr., Falsey, A.R., Kitchin, N., Absalon, J., Gurtman, A., Lockhart, S., Neuzil, K., Mulligan, M.J., Bailey, R., et al. (2020). Safety and Immunogenicity of Two RNA-Based Covid-19 Vaccine Candidates. N Engl J Med 383, 2439-2450.
75. Wang, H., Zhang, Y., Huang, B., Deng, W., Quan, Y., Wang, W., Xu, W., Zhao, Y., Li, N., Zhang, J., et al. (2020). Development of an Inactivated Vaccine Candidate, BBIBP-CorV, with Potent Protection against SARS-CoV-2. Cell 182, 713-721 e719.
76. P. Wang, M. S. Nair, L. Liu, S. Iketani, Y. Luo, Y. Guo, M. Wang, J. Yu, B. Zhang, P. D. Kwong, B. S. Graham, J. R. Mascola, J. Y. Chang, M. T. Yin, M. Sobieszczyk, C. A. Kyratsous, L. Shapiro, Z. Sheng, Y. Huang, D. D. Ho, Antibody resistance of SARS-CoV-2 variants B.1.351 and B.1.1.7. Nature 593, 130-135 (2021).
77. Wimmer, E., Mueller, S., Tumpey, T.M., and Taubenberger, J.K. (2009). Synthetic viruses: a new opportunity to understand and prevent viral disease. Nature biotechnology 27, 1163-1172.
78. Wölfel, R., Corman, V.M., Guggemos, W., Seilmaier, M., Zange, S., Müller, M.A., Niemeyer, D., Jones, T.C., Vollmar, P., Rothe, C., et al. (2020). Virological assessment of hospitalized patients with COVID-2019. Nature 581, 465-469.
79. Wu, A., Peng, Y., Huang, B., Ding, X., Wang, X., Niu, P., Meng, J., Zhu, Z., Zhang, Z., Wang, J., et al. (2020). Genome Composition and Divergence of the Novel Coronavirus (2019-nCoV) Originating in China. Cell Host Microbe 27, 325-328.
80. M. Yuan, D. Huang, C. D. Lee, N. C. Wu, A. M. Jackson, X. Zhu, H. Liu, L. Peng, M. J. van Gils, R. W. Sanders, et al., Structural and functional ramifications of antigenic drift in recent SARS-CoV-2 variants. Science, (2021).
81. Zhang, Y., Zeng, G., Pan, H., Li, C., Hu, Y., Chu, K., Han, W., Chen, Z., Tang, R., Yin, W., et al. (2021). Safety, tolerability, and immunogenicity of an inactivated SARS-CoV-2 vaccine in healthy adults aged 18-59 years: a randomised, double-blind, placebo-controlled, phase 1/2 clinical trial. Lancet Infect Dis 21, 181-192.
82. Zhou, F., Yu, T., Du, R., Fan, G., Liu, Y., Liu, Z., Xiang, J., Wang, Y., Song, B., Gu, X., et al. (2020a). Clinical course and risk factors for mortality of adult inpatients with COVID-19 in Wuhan, China: a retrospective cohort study. Lancet 395, 1054-1062.
83. Zhou, P., Yang, X.L., Wang, X.G., Hu, B., Zhang, L., Zhang, W., Si, H.R., Zhu, Y., Li, B., Huang, C.L., et al. (2020b). A pneumonia outbreak associated with a new coronavirus of probable bat origin. Nature 579, 270-273.
84. Zhu, F.C., Guan, X.H., Li, Y.H., Huang, J.Y., Jiang, T., Hou, L.H., Li, J.X., Yang, B.F., Wang, L., Wang, W.J., et al. (2020). Immunogenicity and safety of a recombinant adenovirus type-5-vectored COVID-19 vaccine in healthy adults aged 18 years or older: a randomised, double-blind, placebo-controlled, phase 2 trial. Lancet 396, 479-488.
85. Zimmer, C., Corum, J., and Wee, S.-L. (2021). Coronavirus Vaccine Tracker. The New York Times.
86. Zou, L., Ruan, F., Huang, M., Liang, L., Huang, H., Hong, Z., Yu, J., Kang, M., Song, Y., Xia, J., et al. (2020). SARS-CoV-2 Viral Load in Upper Respiratory Specimens of Infected Patients. N Engl J Med 382, 1177-1179.
87. Pons-Salort M, Burns CC, Lyons H, Blake IM, Jafari H, Oberste MS, Kew OM, Grassly NC. 2016. Preventing Vaccine-Derived Poliovirus Emergence during the Polio Endgame. PLoS Pathog 12:e1005728.
88. Bull JJ, Smithson MW, Nuismer SL. 2018. Transmissible Viral Vaccines. Trends Microbiol 26:6-15.
89. Layman NC, Tuschhoff BM, Nuismer SL. 2021. Designing transmissible viral vaccines for evolutionary robustness and maximum efficiency. Virus Evol 7:veab002.
90. Nuismer SL, May R, Basinski A, Remien CH. 2018. Controlling epidemics with transmissible vaccines. PLoS One 13:e0196978.
91. Smithson MW, Basinki AJ, Nuismer SL, Bull JJ. 2019. Transmissible vaccines whose dissemination rates vary through time, with applications to wildlife. Vaccine 37:1153-1159.
92. Burns CC, Diop OM, Sutter RW, Kew OM. 2014. Vaccine-derived polioviruses. J Infect Dis 210 Suppl 1:S283-93.
93. Ming LC, Hussain Z, Yeoh SF, Koh D, Lee KS. 2020. Circulating vaccine-derived poliovirus: a menace to the end game of polio eradication. Global Health 16:63.
94. Combelas N, Holmblat B, Joffret ML, Colbere-Garapin F, Delpeyroux F. 2011. Recombination between poliovirus and coxsackie A viruses of species C: a model of viral genetic plasticity and emergence. Viruses 3:1460-84.
95. Lee SW, Markham PF, Coppo MJ, Legione AR, Markham JF, Noormohammadi AH, Browning GF, Ficorilli N, Hartley CA, Devlin JM. 2012. Attenuated vaccines can recombine to form virulent field viruses. Science 337:188.
96. Kamboj M, Sepkowitz KA. 2007. Risk of transmission associated with live attenuated vaccines given to healthy persons caring for or residing with an immunocompromised patient. Infect Control Hosp Epidemiol 28:702-7.
97. Nouailles G, Adler JM, Pennitz P, Peidli S, Alves GT, Baumgart M, Bushe J, Voss A, Langenhagen A, Pott F, Kazmierski J, Goekeri C, Simmons S, Xing N, Langner C, Vidal RM, Abdelgawad A, Herwig S, Cichon G, Niemeyer D, Drosten C, Goffinet C, Landthaler M, Blüthgen N, Wu H, Witzenrath M, Gruber AD, Praktiknjo SD, Osterrieder N, Wyler E, Kunec D, Trimpert J. 2022. A live attenuated vaccine confers superior mucosal and systemic immunity to SARS-CoV-2 variants. bioRxiv doi:10.1101/2022.05.16.492138:2022.05.16.492138.
98. Hoffmann M, Pohlmann S. 2021. How SARS-CoV-2 makes the cut. Nat Microbiol 6:828-829.
99. Koch J, Uckeley ZM, Doldan P, Stanifer M, Boulant S, Lozach PY. 2021. TMPRSS2 expression dictates the entry route used by SARS-CoV-2 to infect host cells. EMBO J 40:e107821.
100. Johnson BA, Xie X, Bailey AL, Kalveram B, Lokugamage KG, Muruato A, Zou J, Zhang X, Juelich T, Smith JK, Zhang L, Bopp N, Schindewolf C, Vu M, Vanderheiden A, Winkler ES, Swetnam D, Plante JA, Aguilar P, Plante KS, Popov V, Lee B, Weaver SC, Suthar MS, Routh AL, Ren P, Ku Z, An Z, Debbink K, Diamond MS, Shi P-Y, Freiberg AN, Menachery VD. 2021. Loss of furin cleavage site attenuates SARS-CoV-2 pathogenesis. Nature 591:293-299.
101. Lau SY, Wang P, Mok BW, Zhang AJ, Chu H, Lee AC, Deng S, Chen P, Chan KH, Song W, Chen Z, To KK, Chan JF, Yuen KY, Chen H. 2020. Attenuated SARS-CoV-2 variants with deletions at the S1/S2 junction. Emerg Microbes Infect 9:837-842.
102. Peacock TP, Goldhill DH, Zhou J, Baillon L, Frise R, Swann OC, Kugathasan R, Penn R, Brown JC, Sanchez-David RY, Braga L, Williamson MK, Hassard JA, Staller E, Hanley B, Osborn M, Giacca M, Davidson AD, Matthews DA, Barclay WS. 2021. The furin cleavage site in the SARS-CoV-2 spike protein is required for transmission in ferrets. Nature Microbiology 6:899-909.
103. Sasaki M, Toba S, Itakura Y, Chambaro HM, Kishimoto M, Tabata K, Intaruck K, Uemura K, Sanaki T, Sato A, Hall WW, Orba Y, Sawa H. 2021a. SARS-CoV-2 Bearing a Mutation at the S1/S2 Cleavage Site Exhibits Attenuated Virulence and Confers Protective Immunity. mBio 12:e0141521.
104. Liu Z, Zheng H, Lin H, Li M, Yuan R, Peng J, Xiong Q, Sun J, Li B, Wu J, Yi L, Peng X, Zhang H, Zhang W, Hulswit RJG, Loman N, Rambaut A, Ke C, Bowden TA, Pybus OG, Lu J. 2020. Identification of Common Deletions in the Spike Protein of Severe Acute Respiratory Syndrome Coronavirus 2. J Virol 94.
105. Sasaki M, Uemura K, Sato A, Toba S, Sanaki T, Maenaka K, Hall WW, Orba Y, Sawa H. 2021b. SARS-CoV-2 variants with mutations at the S1/S2 cleavage site are generated in vitro during propagation in TMPRSS2-deficient cells. PLoS Pathog 17:e1009233.
106. Klimstra WB, Tilston-Lunel NL, Nambulli S, Boslett J, McMillen CM, Gilliland T, Dunn MD, Sun C, Wheeler SE, Wells A, Hartman AL, McElroy AK, Reed DS, Rennick LJ, Duprex WP. 2020. SARS-CoV-2 growth, furin-cleavage-site adaptation and neutralization using serum from acutely infected hospitalized COVID-19 patients. J Gen Virol 101:1156-1169.
107. Wong YC, Lau SY, Wang To KK, Mok BWY, Li X, Wang P, Deng S, Woo KF, Du Z, Li C, Zhou J, Chan JFW, Yuen KY, Chen H, Chen Z. 2021. Natural Transmission of Bat-like Severe Acute Respiratory Syndrome Coronavirus 2 Without Proline-Arginine-Arginine-Alanine Variants in Coronavirus Disease 2019 Patients. Clin Infect Dis 73:e437-e444.
108. Ogando NS, Dalebout TJ, Zevenhoven-Dobbe JC, Limpens R, van der Meer Y, Caly L, Druce J, de Vries JJC, Kikkert M, Barcena M, Sidorov I, Snijder EJ. 2020. SARS-coronavirus-2 replication in Vero E6 cells: replication kinetics, rapid adaptation and cytopathology. J Gen Virol 101:925-940.
109. Davidson AD, Williamson MK, Lewis S, Shoemark D, Carroll MW, Heesom KJ, Zambon M, Ellis J, Lewis PA, Hiscox JA, Matthews DA. 2020. Characterisation of the transcriptome and proteome of SARS-CoV-2 reveals a cell passage induced in-frame deletion of the furin-like cleavage site from the spike glycoprotein. Genome Med 12:68.
110. Walls AC, Park YJ, Tortorici MA, Wall A, McGuire AT, Veesler D. 2020. Structure, Function, and Antigenicity of the SARS-CoV-2 Spike Glycoprotein. Cell 183:1735.
111. Hoffmann M, Kleine-Weber H, Pohlmann S. 2020. A Multibasic Cleavage Site in the Spike Protein of SARS-CoV-2 Is Essential for Infection of Human Lung Cells. Mol Cell 78:779-784 e5.
112. Peacock TP, Goldhill DH, Zhou J, Baillon L, Frise R, Swann OC, Kugathasan R, Penn R, Brown JC, Sanchez-David RY, Braga L, Williamson MK, Hassard JA, Staller E, Hanley B, Osborn M, Giacca M, Davidson AD, Matthews DA, Barclay WS. 2021. The furin cleavage site in the SARS-CoV-2 spike protein is required for transmission in ferrets. Nat Microbiol 6:899-909.
113. Go 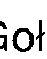 awski, M, Lewandowski, P, Jab 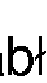 ońska, I, & Delijewski, M. 2022. The Reassessed Potential of SARS-CoV-2 Attenuation for COVID-19 Vaccine Development-A Systematic Review. Viruses, 14: 991.

## Claims

1. A polynucleotide encoding
a) severe acute respiratory syndrome coronavirus 2 (SARS-CoV-2) spike protein; and
b) optionally at least one non-structural SARS-CoV-2 protein selected from the group consisting of non-structural protein 7, non-structural protein 8, non-structural protein 9, non-structural protein 10, non-structural protein 11, non-structural protein 12, an endoribonuclease, and a 2'-O-methyltransferase,
wherein the polynucleotide comprises at least one sequence part comprising codon-pair deoptimizations in comparison to the SARS-CoV-2 genome,
wherein the polynucleotide further comprises a furin cleavage site modification, wherein the furin cleavage site modification results in a loss of a furin cleavage site being naturally present in the SARS-CoV-2 genome.

2. The polynucleotide of claim 1, wherein the furin cleavage site modification is an at least partial deletion of a furin cleavage site being naturally present in the SARS-CoV-2 genome.

3. The polynucleotide of any one of claims 1 and 2, wherein the polynucleotide comprises a nucleic acid sequence as defined by SEQ ID NO. 6 or a nucleic acid sequence having at least 95 % sequence identity to SEQ ID NO. 6.

4. The polynucleotide of any one of claims 1 and 2, wherein the polynucleotide comprises a nucleic acid sequence as defined by SEQ ID NO. 8 or a nucleic acid sequence having at least 95 % sequence identity to SEQ ID NO. 8.

5. The polynucleotide of any one of claims 1 and 2, wherein the polynucleotide comprises a nucleic acid sequence as defined by SEQ ID NO. 10, a nucleic acid sequence having at least 95 % sequence identity to SEQ ID NO. 10, a nucleic acid sequence as defined by SEQ ID NO. 15, a nucleic acid sequence having at least 95 % sequence identity to SEQ ID NO. 15, a nucleic acid sequence as defined by SEQ ID NO. 16, a nucleic acid sequence having at least 95 % sequence identity to SEQ ID NO. 16, a nucleic acid sequence as defined by SEQ ID NO. 17, or a nucleic acid sequence having at least 95 % sequence identity to SEQ ID NO. 17.

6. The polynucleotide of any one of the preceding claims, wherein the furin cleavage site modification comprises a deletion of the nucleotides encoding an amino acid sequence XRRA, wherein X denotes P, R or H.

7. The polynucleotide of any one of the preceding claims, wherein the furin cleavage site modification effects that an expression of the polynucleotide results in a protein in which at least 5 consecutive amino acids of the naturally expressed protein are replaced by a single amino acid.

8. The polynucleotide of any one of the preceding claims, wherein the furin cleavage site modification consists of or comprises a deletion of a nucleic acid sequence as defined by SEQ ID NO. 18 or a nucleic acid sequence having at least 95 % sequence identity to SEQ ID NO. 18.

9. A live attenuated severe acute respiratory syndrome coronavirus 2 (SARS-CoV-2) comprising the polynucleotide according to any one of claims 1 to 8.

10. The live attenuated SARS-CoV-2 according to claim 9, wherein the SARS-CoV-2 has a nucleic acid sequence as defined by SEQ ID NO. 19, a nucleic acid sequence having at least 98 % sequence identity to SEQ ID NO. 19, a nucleic acid sequence as defined by SEQ ID NO. 20, or a nucleic acid sequence having at least 98 % sequence identity to SEQ ID NO. 20.

11. A pharmaceutical composition comprising the live attenuated SARS-CoV-2 according to any one of claims 9 to 10.

12. Pharmaceutical composition according to claim 11 for use as vaccine.

13. A vector comprising the polynucleotide according to any one of claims 1 to 8.

14. A host cell comprising the polynucleotide according to any one of claims 1 to 8.

15. A method for production of a virus, the method comprising the steps of:
a) culturing a host cell according to claim 14; and
b) isolating a virus, wherein the virus is a live attenuated SARS-CoV-2.
